(19)

Europäisches
Patentamt
European
Patent Office
Office européen
des brevets

(11) **EP 4 086 337 A1**

(12) **EUROPEAN PATENT APPLICATION**

(43) Date of publication:
**09.11.2022 Bulletin 2022/45**

(21) Application number: **21172578.3**

(22) Date of filing: **06.05.2021**

(51) International Patent Classification (IPC):
**C12N 1/20** *(2006.01)* **A61K 35/74** *(2015.01)*

(52) Cooperative Patent Classification (CPC):
**C12N 1/20; A61K 35/74**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Maat Pharma**
**69007 Lyon (FR)**

(72) Inventors:
• **AFFAGARD, Hervé**
**SAINTE FOY-LES-LYON (FR)**
• **SCHWINTNER, Carole**
**LYON (FR)**
• **PRESTAT, Emmanuel**
**GREZIEU-LA-VARENNE (FR)**

(74) Representative: **Santarelli**
**49, avenue des Champs-Elysées**
**75008 Paris (FR)**

(54) **METHOD OF PREDICTING AND THEN PRODUCING A MIX OF MICROBIOTA SAMPLES**

(57)      Prediction of a mix of complex communities of microorganisms includes a linear prediction, e.g. matrix-based, that is corrected using an interaction model, e.g. a matrix, learnt from reference true mix profiles and corresponding reference linear-predicted profiles. Reverse prediction makes it possible to determine a mix of samples to be made given a target mix profile.

Figure 1

EP 4 086 337 A1

**Description**

**FIELD OF THE INVENTION**

The present invention concerns the mixing or "pooling" of complex communities of microorganisms, or microbiotas, and more particularly methods and devices using a learnt model, for example a matrix-based predictor, linking the individual profiles or compositions of initial microbiota samples with the profiles of resulting mixes thereof.

**BACKGROUND AND PRIOR ART**

Complex communities of microorganisms, also known as microbiotas, play a key role in health and diseases. In particular, it has been discovered that the administration or transplantation of a complex community of microorganisms, for instance via Fecal Microbiota Transplantation (FMT), may treat infections and diseases.

In case of administration or transplantation of a complex community of microorganisms, it is important for the administrated or transplanted sample to have an appropriate profile in terms of viability and diversity of microorganisms such as bacteria, archaea, viruses, phage, protozoa and/or fungi.

Some administration and transplantation methods are often empirical and take no particular precaution to ensure the diversity of the microorganisms present in the used samples, or to best preserve the viability of the microorganisms.

Furthermore, samples collected from donors may not offer satisfactory profiles of complex communities of microorganisms for an efficient treatment.

Mixes of complex microorganism community samples collected from several donors have thus been considered to increase the diversity of the samples that can be used as inocula for administration or transplantation.

To test various mixes, the mixing of samples is actually performed randomly, and resulting products are then sequenced in order to obtain final mix profiles, from which curative and treatment properties are inferred. This test-based approach has some drawbacks. In particular, it consumes rare material given the harsh difficulties in obtaining samples from donors and takes several weeks to be completed due to sequencing analysis time.

Prediction of the mix composition, i.e. of the profile of the mix product, has thus been contemplated.

A simple way to predict the mix composition from the individual profiles of the complex microbial communities used as starting material consists in applying a linear prediction for each profiling feature: for instance, by summing the relative abundances of said profiling feature in the individual profiles after weighting them by the ratio of the corresponding complex communities in the mix.

However, some shifts or drifts between such linear-predicted profiles and the true profiles (obtained by profiling the mix result products) were observed. Hence, the linear prediction of profiles is thus considered as being a naive approach.

An assumption of the inventors is that the shifts may result from some important and quick adjustments of the microorganisms due to their interactions in the shared environment, for instance an adaptation to new conditions of the shared environment or a competition between microorganisms.

There is thus a need to perform accurate predictions to conduct the pooling in a way that guaranties the delivery of a precise complex community product composition e.g. with expected treatment efficacy.

**SUMMARY OF THE INVENTION**

The present invention seeks to overcome some of the foregoing concerns by computer-aided designing these shifts when predicting mix compositions, with a view of driving, controlling or directing an actual mixing of microbiota samples for use thereof in, *inter alia,* administration or transplantation methods.

In this respect, the present invention proposes a computer-aided method of predicting a mix composition resulting from the mixing of complex microorganism community samples belonging to an initial sample collection, the method comprising:

predicting, using a linear approach, an intermediary mix profile for a mix of selected complex microorganism community samples, and
correcting the intermediary mix profile into a predicted mix profile, using an interaction model learnt from reference linear-predicted mix profiles and corresponding reference true mix profiles.

In particular, the predicted mix profile may be used to control actual picking and mixing of complex microorganism community samples from the initial sample collection to obtain a mix result product. Picking samples may simply mean taking or retrieving appropriate and sufficient quantities of the samples from the initial collection. The picking may be made either manually by an operator or automatically by controlled robots.

It turns out that the present invention also provides a method of producing a complex microorganism community product, comprising:

selecting complex microorganism community samples from an initial sample collection,
using the above prediction method to predict a mix profile resulting from the mixing of the selected samples,
comparing the predicted mix profile to a selection criterion, for example the sufficient presence of taxa of interest or any target mix profile, and
depending on the outcome of the comparing, actually picking and mixing the selected samples to obtain a mix result product. The selected samples are preferably mixed using the relative abundancies used for the prediction.

Of course, should the outcome of the comparing be deceiving, no actual mixing may be performed, but another selection of samples may be made to predict another mix profile with the teachings of the invention. Thus, a plurality of sets of selected samples may be successively considered. Next, for each set, the using and comparing steps are performed and then the actual picking and mixing are performed depending on the outcome of their comparing.

Conversely, the present invention also proposes a reverse approach with a computer-aided method of determining a target set of complex microorganism community samples in an initial sample collection given a target mix profile representing a target mix result product, the method comprising:

selecting candidate sets of complex microorganism community samples from the initial sample collection,
for each candidate set selected, using the above prediction method to predict a mix profile resulting from the mixing of the samples of the selected candidate set,
comparing the predicted mix profiles to the target mix profile to choose one candidate set as the target set.

The target mix profile may be general, i.e. quantifying each profiling feature considered, or be specific to one or some profiling features, e.g. defining some feature specifications such as the presence or absence of one (or more) profiling feature(s) and/or its relative abundance or quantity or ranges of quantities, or defining a minimum level of diversity for instance in terms of a number of profiling features with minimum relative abundancies. The target mix profile may thus be a set of profiles scanning various possible values for a given feature specification.

The target set of samples may then be used to control actual picking and mixing of complex microorganism community samples from the initial sample collection to obtain a mix result product function of the target mix profile (it may have the target mix profile or be close to it given approximations).

It turns out that the present invention also provides a method of producing a complex microorganism community product having a target mix profile representing a target mix result product, comprising:

selecting, using the above determining method, a target set of complex microorganism community samples belonging to an initial sample collection given the target mix profile, and
actually picking and mixing the samples of the selected target set to obtain a mix result product.

The present invention advantageously makes it possible to instantaneously simulate various mix compositions at low cost, in particular without consuming any actual material (samples of the initial sample collection).

It further allows efficient sets of complex microorganism community samples to be found with a view of obtaining a mix result product that meets mix criteria, for instance a target community profile or composition adapted to cure a disease.

Hence a pooling strategy can be defined ahead of a production routine, depending on the needs of the intended use (e.g. therapeutic, prophylactic, environmental, ...).

The mix result product so obtained can then be administrated or transplanted into a human or animal body or to plants as a fertilizer or even to environment media, including water, soil and subsurface material, e.g., for treating contamination via bioremediation.

Preferably, Microbiome Ecosystem Therapy products can be produced using the above methods.

Correlatively, the invention also provides a computer device comprising at least one microprocessor configured for carrying out the steps of any of the above methods. The computer device may thus be configured to emit a signal to control a mixing device to actually pick and mix complex microorganism community samples from the initial sample collection to obtain a mix result product.

Optional features of embodiments of the invention are defined in the appended claims. Some of these features are explained here below with reference to a method, while they can be transposed into device features.

In some embodiments, predicting the intermediary mix profile includes computing a matrix product between a first matrix defining the mix in terms of proportions of the complex microorganism community samples of the initial sample collection and a second matrix defining the individual profiles of the complex microorganism community samples. The second matrix, denoted A below, is defined by the initial sample collection available.

In some embodiments, correcting the intermediary mix profile includes computing a matrix product between a matrix representing the intermediary mix profile and a square interaction matrix of the learnt interaction model. Here, the interaction model may be the square interaction matrix learnt from the reference linear-predicted mix profiles and the corresponding reference true mix profiles.

Using matrices to perform the prediction of sample mixes advantageously allows a large number of profiling features to be taken into account and quick computations to obtain one or more predicted mix profiles for mix result product or products.

In some embodiments, the predicting method further comprises clipping each negative value in the predicted mix profile, i.e. the negative values are set to 0. This is to correct theoretical predictions (for instance relative abundance becomes negative) to nature reality.

In some embodiments, the predicting method further comprises normalizing to 1 a sum of relative abundancies of profiling features defining the predicted mix profile. Again, this aims at normalizing theoretical predictions to nature reality. This is to have true relative abundancies, the sum of which represents an entire composition.

It is also expected that a profiling feature not present in the selected samples (that are mixes) should not be present in the predicted mix profile. Hence, non-zero abundances in the predicted mix profile for profiling features initially not present in the selected samples are set to zero.

In some embodiments regarding the reverse approach, determining the set of samples may include determining a relative abundance of each sample within the set. In other words, the reverse prediction aims at obtaining the relative proportions of the complex microorganism community samples to be mixed together.

In other embodiments regarding the reverse approach, comparing the predicted mix profiles to the target mix profile includes computing a distance between each predicted mix profile and the target mix profile and selecting, as target set, the candidate set having the lowest distance.

In some embodiments, a profile of a complex community of microorganisms (sample or mix) includes relative abundancies of profiling features in the complex community of microorganisms.

In specific embodiments, the relative abundancies are representative of mass or volume proportions of the profiling features in the complex community of microorganisms.

In some embodiments, profiling features forming a profile of a complex community of microorganisms include one or more features from taxa, genes, antibiotic resistance genes, functions, metabolite traits, and metabolite and protein production, preferably include taxa.

In some embodiments, an individual profile of a complex microorganism community sample is obtained using a profiling technology such as 16S rRNA gene amplicon sequencing, NGS shotgun sequencing, amplicon sequencing other than 16S rRNA gene-based, NGS amplicon-based targeted sequencing, phylochip-based profiling, whole metagenome sequencing (WMS), Polymerase Chain Reaction (PCR) identification, a mass spectrometry (e.g. of LC/MS type or GC/MS type), near-infrared (NIR) spectroscopy, nuclear magnetic resonance (NMR) spectroscopy, preferably using the 16S rRNA gene amplicon sequencing or NGS.

In some embodiments, a profile of a complex community of microorganisms defines profiling features with respect to one or more microorganisms present in the complex community of microorganisms from bacteria, archaea, viruses, phage, protozoa and fungi, preferably with respect to bacteria and/or archaea.

In some embodiments, a profile of a complex community of microorganisms defines profiling features that specify relative abundances of microorganisms considered at one or more taxonomic levels from strains, species, genus, families and orders, preferably one or more taxonomic levels from genus, families and orders.

In some embodiments, a profile of a complex community of microorganisms includes relative abundancies, in the complex community of microorganisms, of bacteria and/or archaea taxa considered at a taxonomic level of genus, families and orders.

In some embodiments, a profile of a complex community of microorganisms includes relative abundancies, in the complex community of microorganisms, of bacteria and/or archaea taxa defined by the presence/absence or expression of certain genes and/or functions (e.g., production of butyrate, antibiotic resistance genes, production of enzymes such as organophosphate hydrolases, phosphodiesterases, superoxide dismutases, *etc.,* production of anti-microbial peptides, organophosphate hydrolyases or other enzyme useful in bioremediation processes, ...).

In some embodiments, the initial sample collection comprises samples selected from the group consisting of raw complex microorganism community samples, engineered/processed complex microorganism community samples, artificial complex microorganism community samples (e.g., bacterial consortia obtained by mixing isolated strains) and virtual complex microorganism community samples.

In some embodiments, the initial sample collection includes one or more of faecal, skin, buccal, vaginal, nasal, tumoral, human, animal, plant, water, soil samples. For instance, it may include one or more faecal samples coming from at least one donor, preferably coming from at least two donors.

In some embodiments, the interaction model (e.g. the square interaction matrix) is obtained using machine learning that minimizes a formula function of a difference between

reference predicted mix profiles obtained from the reference linear-predicted mix profiles and the interaction model (preferably, a matrix product is performed with the square interaction matrix), and

the corresponding reference true mix profiles.

The reference data (here profiles) are known as training data for the machine learning process. It is searched to minimize the error between matrix-based predicted profiles and corresponding true profiles, possibly given a regularization term.

In this respect, the formula may add a regularization term, preferably a Ridge-based regularization term, to said difference.

In particular embodiments, the regularization term includes a difference between a square interaction matrix of the interaction model and the identity matrix.

The regularization tends to penalize model solutions too far from the identity. Indeed, it is expected that the interactions between the microorganisms within the mixes are not too substantial, so that model solutions far from the identity are far from biological reality. The regularization term thus avoids such accidental solutions (theoretical solutions due to the particular set of training data) to be obtained.

In some embodiments, negative values of relative abundancies of profiling features in the reference predicted mix profiles are clipped before minimizing the formula.

In some embodiments, the method further comprises normalizing to 1 a sum of relative abundancies of profiling features defining one of the reference predicted mix profiles before minimizing the formula. Preferably, several or all the reference predicted mix profiles are individually normalized to 1 if necessary.

In some embodiments, a reference linear-predicted mix profile is predicted, using a linear approach, from individual profiles of complex microorganism community samples mixed together to produce a reference mix product and the corresponding reference true mix profile is obtained from profiling (e.g. sequencing or 16S rRNA gene amplicon sequencing) the reference mix product.

In some embodiments relating to the producing method, the selection criterion includes one or more from a diversity criterion representative of an increase in profiling feature diversity, a minimum or maximum relative abundance of one or more profiling features, a non-zero relative abundance for one or more specific profiling features or for a minimum number of profiling features, a relative ratio between at least two profiling features, a closeness (or similarity such as minimal distance) to a target mix profile.

In some embodiments, one selected complex microorganism community sample is a virtual sample and the method further comprises actually producing a complex microorganism community sample corresponding to the selected virtual sample from isolated strains and/or complex microorganism community samples. This advantageously allows defining a pooling strategy ahead, without consuming material nor having yet the samples. A bacteria consortium identified as being useful to produce a desired mix result product can then be produced by a mere mixing of isolated strains. Similarly, a sample identified as being useful to produce a desired mix result product can be produced by mixing one or several of isolated strains with one or several samples, resulting in an engineered sample enriched in the desired strains.

Another aspect of the invention relates to a non-transitory computer-readable medium storing a program which, when executed by a microprocessor or computer system in a device, causes the device to perform any method as defined above.

At least parts of the methods according to the invention may be computer implemented. Accordingly, the present invention may take the form of an entirely hardware embodiment, an entirely software embodiment (including firmware, resident software, micro-code, etc.) or an embodiment combining software and hardware aspects that may all generally be referred to herein as a "circuit", "module" or "system". Furthermore, the present invention may take the form of a computer program product embodied in any tangible medium of expression having computer usable program code embodied in the medium.

Since the present invention can be implemented in software, the present invention can be embodied as computer readable code for provision to a programmable apparatus on any suitable carrier medium. A tangible carrier medium may comprise a storage medium such as a hard disk drive, a magnetic tape device or a solid-state memory device and the like. A transient carrier medium may include a signal such as an electrical signal, an electronic signal, an optical signal, an acoustic signal, a magnetic signal or an electromagnetic signal, e.g. a microwave or RF signal.

## BRIEF DESCRIPTION OF THE FIGURES

**Figure 1** illustrates a complex microorganism community mixing platform implementing embodiments of the present invention;

**Figure 1a** illustrates the behavior of an error measurement depending on an hyper-parameter of a regularization term when modelizing;

**Figure 2** illustrates, using a flowchart, general steps of producing a mix result product, including predicting mix profiles, according to embodiments of the invention;

**Figure 3** illustrates, using a flowchart, general steps of determining and then producing a mix result product given a target mix profile, according to embodiments of the invention;

**Figure 4** shows a schematic representation a computer device in accordance with embodiments of the present invention;

**Figures 5a, 5b** and **5c** illustrate results of a first experiment of the present invention, based on mixing native complex community samples of microorganisms;

**Figures 6a, 6b** and **6c** illustrate other experimental results of the present invention, based on mixing fermented complex community samples of microorganisms;

**Figures 7a** and **7b** illustrate yet other experimental results of the present invention, mixing native and fermented samples;

**Figure 8** illustrate a collection of sample profiles used in a second experiment of the present invention;

**Figures 9a** and **9b** illustrate results of the second experiment seeking to find a mix composition to obtain a mix product close enough to a target mix product; and

**Figure 10** illustrates the similarities between actual mixes or actual and predicted mixes when comparing the target mix product and the best predicted mix product shown in **Figures 9a** and **9b.**

## DETAILED DESCRIPTION

The present invention concerns the mixing or "pooling" of complex communities of microorganisms, or "microbiotas" or "microbiota samples". It is more particularly directed to methods and devices using a learnt predictor model linking the individual profiles or compositions of initial complex microorganism community samples with the profiles of resulting mixes thereof.

As used herein, the expressions "microbiota", "microbiota composition" and "complex community of microorganisms" can be used interchangeably to refer to any population of microorganisms comprising a high number of microorganisms of different species which live together and potentially in interaction. Microorganisms possibly present in a complex community of microorganisms include yeasts, bacteria, archae, virus, fungi, algae, phages, and any protozoa of different origins such as soil, water, vegetal, animal, or human origins.

Microbiotas according to the present text include naturally occurring complex communities of microorganisms (such as, for example, gut microbiota, i.e., the population of microorganisms living in the intestine of an animal), as well as "engineered complex communities of microorganisms", i.e., complex communities resulting from transformation steps such as addition of isolated beneficial strains, treatments to remove potential deleterious microorganisms (e.g., by using rare-cutting endonucleases targeting genes specific for pathobionts), expansion by culture in specific conditions (e.g., fermentation in appropriate medium), etc. "Isolated beneficial strains" herein designate natural strains known to have a beneficial effect in certain conditions (e.g., *Akkermansia muciniphila*), as well as genetically modified strains, including strains in which a potential deleterious gene has been knocked out (for example using a rare-cutting endonuclease such as Cas9) and strains in which a transgene has been introduced (e.g., by the use of a bacteriophage, or the CRISPR system).

Complex communities of microorganisms and microbiotas according to the present text include "raw" or "native" complex communities or microbiotas, i.e., directly obtained from a source, a donor or donors without being treated by post-processing and "processed complex communities of microorganisms", including engineered complex communities or microbiotas and any complex microorganism communities resulting from a treatment on or post-processing of or transformation of one or more natural raw complex microorganism communities (e.g., a complex community or microbiota which been filtered, frozen, thawed and/or lyophilized, and/or which has been extracted, isolated or separated from its initial matrix by techniques well-known for the skilled person such as, for example, those described in WO 2016/170285 and WO 2017/103550).

The expression "samples", "complex microorganism community samples" and "microbiota samples" can be used interchangeably and refer to initial complex communities or microbiotas in the meaning of the invention, i.e. that are available for mixing.

The term "Microbiome Ecosystem Therapy product" herein refers to any composition comprising a complex community of microorganisms (either naturally occurring or engineered, native or processed), provided it is in a form suitable for administration to an individual in need thereof. A Microbiome Ecosystem Therapy aims at modifying an individual's microbiota to obtain a health benefit (e.g., preventing or alleviating the symptoms of a disease, increasing the chances that the individual responds to a treatment, etc.). Typically, a Microbiome Ecosystem Therapy is done by replacing at least part of a dysfunctional and/or damaged ecosystem by a different complex community of microorganisms in a subject in need thereof. Microbiome Ecosystem Therapies include Fecal Microbiota Transplantation (FMT). In the present text, unless specified otherwise, the term "FMT" is broadly used to refer to any kind of Microbiome Ecosystem Therapy.

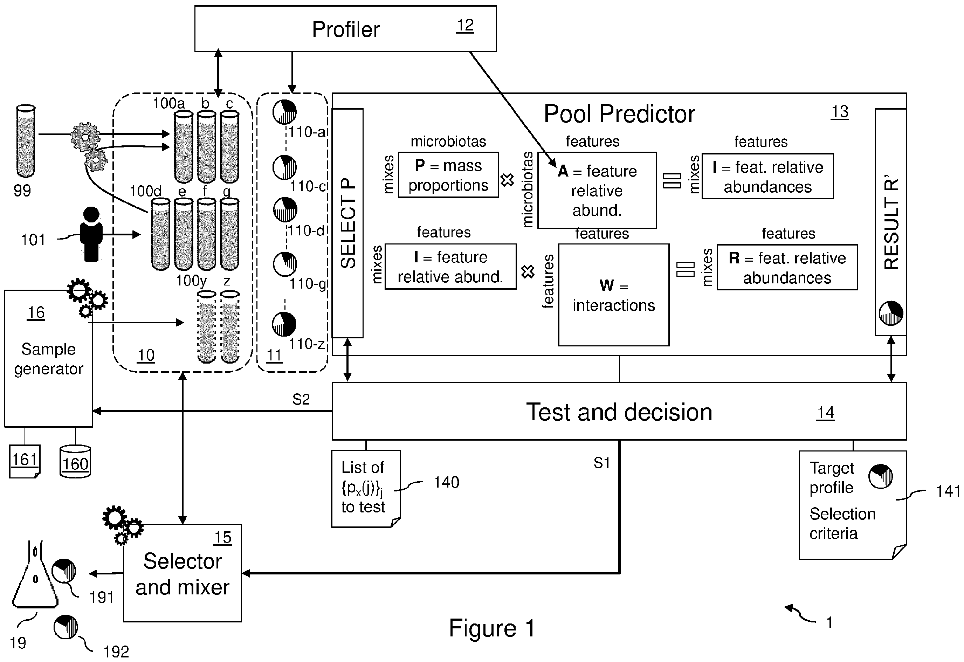

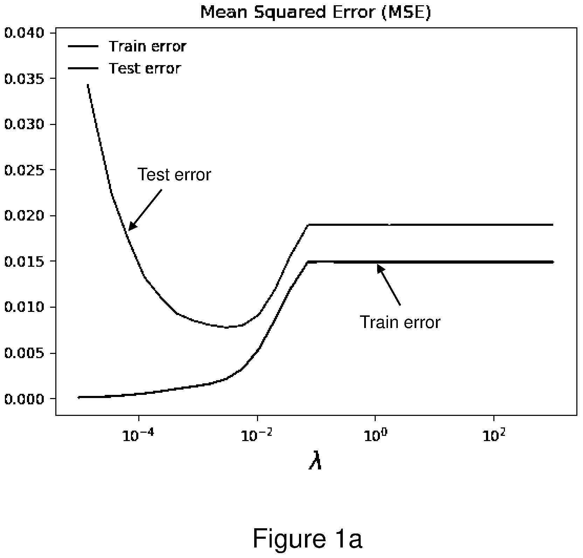

As shown in **Figure 1** illustrating a complex microorganism community mixing platform 1 implementing embodiments of the present invention, samples 100 are available through an initial sample bank or collection 10. Although a single collection or bank is shown, the samples may be stored in a plurality of sub-banks that altogether form collection or bank 10.

The samples of the present invention may comprise or may consist of microorganisms coming from one or more sources and/or from one or more donors 101.

The samples of the present invention may come from:

- a single source,
- at least two sources,
- a single donor,
- at least two donors,
- a single source and a single donor,
- a single source and at least two donors,
- at least two sources and a single donor, or
- at least two sources and at least two donors,

As used herein, the term "source" refers to any environment from where the sample comes from such as a soil, water, parts of a vegetal, parts of animal body or fluids or parts of human body or fluids. In case of a human or an animal, the source may refer to any part of the body (skin, nasal mucosa, ...) or to body fluids such as the content of the intestine (e.g., a stool sample).

As used herein, the term "donor" refers to a vegetal, a physical location (for sources such as soil or water), an animal or a human, preferably a human.

The donors may be pre-selected according to the method and criteria described in the prior art, such as for example in WO2019/171012 A1.

In the example shown, some samples, referenced 100d, 100e, 100f, 100g, are raw complex communities of microorganisms or microbiotas, i.e., directly obtained from a donor or donors without being treated by post-processing.

Other samples, referenced 100a, 100b, 100c, are "processed samples", i.e., engineered complex microorganism communities resulting from a treatment on or post-processing of or transformation of one or more natural raw complex communities. As mentioned above the treatment may include filtration, centrifugation, fermentation, freezing, freeze-drying the initial complex community, and even mixing of initial complex communities, but also treatments aimed at isolating spores and spore forming bacteria such as the use of ethanol, chloroform or heat.

As shown, an initial complex community may be one sample 100d, 100e, 100f, 100g belonging to the initial sample collection 10 or be an external sample 99.

The initial sample collection 10 may include one or more samples from any source (faecal, skin, nasal, buccal, vaginal, tumoral...) of any origin (human, animal, plant, soil, ...), preferably one or more faecal samples coming from at least one donor, preferably coming from at least two donors.

According to a particular embodiment, the samples of the collection 10 include faeces samples.

Faeces samples collected from donors may be controlled according to the method and qualitative criteria described in the prior art, such as for example in WO2019/171012 A1. For example, the qualitative criteria of the sample may comprise sample consistency between 1 and 6 on the Bristol scale; absence of blood and urine in the sample; and/or absence of specific bacteria, parasites and/or virus, as described in WO2019/171012 A1.

Faeces samples may be collected according to any method described the prior art, such as for example in WO2016/170285 A1, WO2017/103550 A1 and/or WO2019/171012 A1. Preferably, the samples may be collected and then placed in anaerobic conditions. For example, as described in WO2016/170285 A1, WO2017/103550 A1 and/or WO2019/171012 A1, within 5 minutes following taking of the sample, the samples may be placed in an oxygen-tight collecting device.

The samples may be prepared according to the methods described in the prior art, such as for example in WO2016/170285 A1, WO2017/103550 A1 and/or WO2019/171012 A1.

All samples 100a-100g shown in the Figure are actual samples stored in at least one bank.

Samples 100y-100z represented with dotted lines are theoretical samples that are not actually collected from a donor or produced, hence not actually stored in the storage bank or banks 10. As explained below, these "virtual" samples 100y-100z are depicted to illustrate theoretical complex community profiles 110z imagined by an entity, for instance a computer, an operator, a researcher, and so on.

The initial sample collection 10 may comprise only native samples 100d-100g, or may comprise only processed samples 100a-100c, or may comprise only virtual samples 100y-100z, or any combination thereof.

A first object of the present invention relates to the prediction of a mix composition resulting from the mixing of samples 100a-100z belonging to the initial sample collection 10. The prediction is two-fold:

predicting, using a linear approach, an intermediary mix profile for a mix of selected complex microorganism community samples, and

correcting the intermediary mix profile into a predicted mix profile, using an interaction model learnt from reference linear-predicted mix profiles and corresponding reference true mix profiles. The interaction model is preferably a squared interaction matrix learnt from the reference linear-predicted mix profiles and the corresponding reference true mix profiles.

The present inventors have surprisingly found that a learnt interaction model, and more particularly the matrix-based method, provides accurate prediction results once the interaction model or matrix is learnt, hence giving relevant hints to a final product without consuming any material of the initial sample collection.

As the prediction can be computer-implemented, the predicted mix profiles can be quickly obtained despite a large number of mixes to predict, a large number of samples available in the initial sample collection 10, and a large number of features profiling the complex communities of microorganisms (samples and mixes).

A second object of the present invention relates to a reverse operation where a target set of samples is determined from the initial sample collection 10 given a target mix profile representing a target mix result product. The target mix result product may for instance represent a desired complex community of microorganisms having curative properties with respect to a disease or infection.

The reverse operation includes:

successively selecting candidate sets of complex microorganism community samples from the initial sample collection,

for each candidate set successively selected, using the above prediction method to predict a mix profile resulting from the mixing of the samples of the selected candidate set,

comparing the predicted mix profiles to the target mix profile to choose one candidate set as the target set.

Both prediction operation and reverse operation can be used to actually produce a mix result product.

With respect to the prediction operation, the predicted mix profile may then be compared to a selection criterion, for instance the sufficient presence of taxa of interest. Then, depending on the outcome of the comparing, the selected samples are retrieved and actually mixed to obtain the mix result product. The predicted mix profile can thus be used to control actual picking and mixing of samples from the initial sample collection to obtain the mix result product.

The selection criterion may be set function of desired properties for the mix result product.

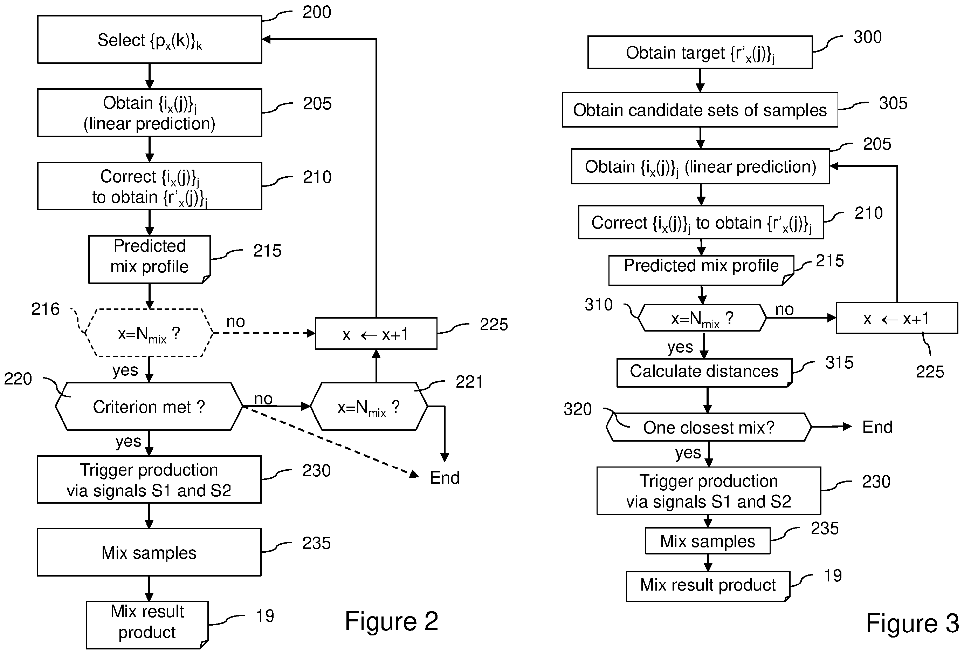

Such an approach, including the interaction-model-based or matrix-based prediction, is illustrated in **Figure 2**, described with more details below.

With respect to the reverse operation, a target set of samples belonging to the initial sample collection is selected using the reverse operation given a target mix profile, corresponding for instance to a mix result product having desired curative properties. Next, the samples of the target set are picked and actually mixed to obtain the desired mix result product. The target set of samples determined by the reverse predicting method can thus be used to control actual picking and mixing of samples from the initial sample collection to obtain a mix result product function of the target mix profile.

Embodiments of such reverse approach using the target mix profile are illustrated below with reference to **Figure 3.**

By "mix" it is meant any actual mixing of samples that results in a new complex community of microorganisms or new microbiota composition. The result is also referred to as mix result product as it may be used for administration or transplantation as described above. The mix result product may for instance be used as an FMT inoculum.

By "profile" it is meant a description of the composition of the complex community of microorganisms or microbiota composition concerned (be it a sample or a mix). A profile for instance specifies the relative abundancies of profiling features in the complex community or microbiota composition. "relative" means that the sum of the abundancies equals to 1. The relative abundancies may be expressed in mass (or weight) or volume proportions of the profiling features in the complex community of microorganisms.

Depending on the application concerned (for instance, in the therapeutic field, depending on the disease targeted and, in the bioremediation field, depending on the pollutants to eliminate), the profiling features may be of different types. Usually, they are selected from a group including taxa, genes, antibiotic resistance genes, functions, and metabolite traits, and metabolite and protein production. A profile may mix profiling features of different types, for instance taxa and antibiotic resistance genes. A particular embodiment considers only taxa to profile a complex community of microorganisms.

Various profiling techniques are known to obtain complex community profiles, including 16S rRNA gene amplicon (i.e. metagenome) sequencing, NGS shotgun sequencing, amplicon sequencing other than 16S rRNA gene-based, NGS amplicon-based targeted sequencing, 18S/ITS gene sequencing, metagenomic sequencing, a phylochip-based profiling, a Polymerase Chain Reaction (PCR) identification, a mass spectrometry (e.g. of LC/MS type or GC/MS type), near-infrared (NIR) spectroscopy and nuclear magnetic resonance (NMR) spectroscopy.

As shown in **Figure 1**, a profiler (or sequencer) 12 is preferably used to provide a profile, e.g. a 16S sequencing, of the actual samples 100a-100g. The corresponding individual profiles so obtained are referenced 110a-110g and form an initial profile collection or bank 11. Of course, the 16S rRNA sequencing is not mandatory and other methods can be used as defined above, alone or in combination, to provide the profiles 110.

The individual profiles, whatever the sequencing technique used, are converted in the same format and stored in a memory (not shown) of a computer as matrices or vectors $a_x$. The coefficient $a_x(j)$ of individual profile 'x' indicates the relative abundance of the profiling feature 'j' in the sample considered.

As mentioned previously, some individual profiles 110z may be artificially built by an operator, e.g. by defining coefficients $a_x(i)$ representing the relative abundances of the profiling features 'j' in a theoretical sample.

The initial profile collection 11 may thus comprise only individual profiles 110d-110g corresponding to native samples 100d-100g, or may comprise only individual profiles 110a-110c corresponding to processed samples 100a-100c, or may comprise only virtual profiles 110y-110z corresponding to virtual samples 100y-100z, or any combination thereof.

Any other profile handled thereafter (e.g. so-called intermediary profiles or mix profiles) follows the same profile format, for instance a vector made of the same profiling features 'j' in the same order.

Preferably, bacterial abundance profiles are obtained, meaning that the profiles specify relative abundancies of profiling features concerning bacteria. More generally, a profile of a complex community of microorganisms may define profiling features with respect to one or more microorganisms present in the complex community (bacteria, archaea, viruses, phage, protozoa and fungi), preferably with respect to bacteria and/or archaea. Of course, profiling features within the same profile may concern different microorganisms as previously listed.

Preferably, genus-based bacterial abundance profiles are obtained, meaning the profiling features describe the relative abundancies of bacteria at genus level in the complex community of microorganisms. More generally, a profile of a complex community of microorganisms may define profiling features that specify relative abundances of microorganisms considered at one or more taxonomic levels from strains, species, genus, families and orders, preferably one or more taxonomic levels from genus, families and orders.

The prediction operation and the reverse operation are conducted by the pool predictor module 13 under the control of the module 14. Module 14, referred to as "test and decision module" or "decision module", drives platform 1 with a view of predicting mix profiles and / or determining a set of samples given a target mix profile and / or producing at least one mix result product.

Modules 13 and 14 are preferably implemented through a computer, having an input/output interface (e.g. keyboard, mouse, screen) to allow an operator to interact with platform 1.

As shown in the Figure, pool predictor 13 is matrix-based and comprises two steps for predicting a result mix profile from initial profiles of samples mixed together.

Matrix A defines the individual profiles of all the samples available in the collection 10. It may be formed by profiler or sequencer 12 or at least by the individual profiles obtained from the profiler. Additionally any virtual individual profile is also added to the matrix.

$$A = \begin{bmatrix} \{a_1(j)\}_j \\ \{a_n(j)\}_j \end{bmatrix}$$

Preferably where j = 1 ... m, with m the number of profiling features considered and n the number of individual profiles 110 in the initial profile collection 11, hence of samples 100 (including the virtual ones) in the initial sample collection 10.

Square matrix W is the interaction matrix defined above modelling the interaction between the microorganisms. A description of the modelling matrix W, including how it is learnt, is provided below with more details. The interaction matrix aims at representing the non-linear interactions between the various profiling features of samples when the latter are mixed together.

The prediction operation comprises a first matrix-based step of predicting, using matrix A, an intermediary mix profile, formed by matrix I, for at least one mix of selected samples: I = P * A, where P is a matrix representing the at least one mix of selected samples from collection 10.

Matrix P may define each mix in terms of mass or volume proportions of the samples of the initial sample

collection.

$$P = \begin{bmatrix} \{p_1(k)\}_k \\ \{p_t(k)\}_k \end{bmatrix}$$

For instance, where $\{p_x(j)\}_j$ defines a mix 'x' with $p_x(k)$ the proportions of sample k (k from 1 to the number Nsamp of samples in collections 10/11). The sum of the proportions equals 1: $\sum_{k=1...Nsamp}(p_x(k)))=1$. Where a sample r is not used in the mix x, $p_x(r)=0$.

The matrix-based approach advantageously allows a varying number of mixes to be predicted together: each line of P defines a mix to predict (hence 't' mixes are defined in the above example), which number 't' can vary from one prediction to the other.

The mixes to test, i.e. a list of $\{p_x(k)\}_k$ may be defined in advance in list 140 of test and decision module 14. Upon starting a new test procedure, module 14 reads list 140 and forms matrix P defined above using one or some or all the mix definitions of the list. Of course, in a variant or in combination, an operator may define at least one new mix on the fly by selecting, on a screen connected to module 14, samples 100 from the collection 10 and by specifying their relative proportions. Hence a mix $\{p_x(k)\}_k$ can be created on the fly.

The prediction operation I = P * A is for instance computer-implemented.

$$I = \begin{bmatrix} \{i_1(j)\}_j \\ \{i_t(j)\}_j \end{bmatrix}$$

Matrix with j=1... m, is obtained defining the linear-predicted or "intermediary" mix profiles for the tested mixes P. Those mix profiles are "naive" predictions because they do not take into account the interactions between the microorganisms when the mix is actually performed.

That is why, according to the invention, the prediction operation comprises a second step of correcting, using the interaction model, in particular interaction matrix W, the intermediary mix profiles, i.e. matrix I, into predicted mix

$$R = \begin{bmatrix} \{r_1(j)\}_j \\ \{r_t(j)\}_j \end{bmatrix} :$$

profiles, represented by matrix : R = I * W, with j=1... m.

Predicted mix profiles can thus be obtained quickly for a varying number of mixes, without consuming any material of collection 10.

It is expected that the relative abundancies $r_x(j)$ are not negative and form together an entire composition (i.e. their sum equals 1 for a given mix 'x'). However, this may not be the case with a matrix product. Embodiments of the invention thus include post-processing result matrix R into R' in order to meet biological constraints.

For instance, each negative value in R is clipped, meaning the negative abundancies are set to 0. Thereafter, the relative abundancies $r_x(j)$ are normalized, i.e. adjusted (using a linear interpolation for instance) into $r'_x(j)$ so that their

$$R' = \begin{bmatrix} \{r'_1(j)\}_j \\ \{r'_k(j)\}_j \end{bmatrix}$$

sum equals 1: $\sum_{j=1...m}(r'_x(j))=1$. The final mix result matrix is the following one where $\{r'_x(j)\}_j$ is a vector representing the predicted mix profile for tested mix x (defined by $\{p_x(k)\}_k$). Optionally, before normalizing, non-zero relative abundancies (non-zero value in R) for profiling features that are absent in the initial samples mixed together (i.e. $a_x(j)$ is zero for all samples x mixed together) are set to zero.

The efficiency of the present method comes from the modelling of the real positive and negative interactions between the microorganisms of mixed samples into a matrix, so-called interaction matrix W. Then a two-step matrix-based process is efficiently used to predict real mix profiles.

Interaction matrix W is learnt for a given set of m profiling features. Should the profiling features be reordered in the profiles, the coefficients of interaction matrix W should be reordered accordingly.

The m profiling features may also evolve over time, for instance because new features are discovered, some features become less meaningful hence they are deleted, and / or some features can be split into more features to be more precise. Evolution in the profiling features may also result from the enhancement of the profiling / sequencing methods and profilers / sequencers 12 that provide new profiling data, as well as the improvements of the bioinformatics method that combines algorithm and reference databases of features.

Different sets of profiling features may also be considered, for instance with respect to different diseases or treatments that are targeted.

The profiling features themselves but also the number of features in the sets can evolve or change.

Hence, each time a new set of profiling features is considered, interaction matrix W can be computed anew, as well as matrix A describing the initial profile collection 11. The computed interaction matrices W may be stored in memory of pool predictor 13 so that they may be reused, should the corresponding set of profiling features be used anew.

Interaction matrix is preferably obtained using machine learning. The machine learning is made using a set of training data. The training data are built from reference mix products 'ref' resulting from a plurality of mixes $\{p_{ref}(k)\}$ of samples k.

An actual reference mix of samples is homogenised during a period of 10 minutes to 3 hours, preferably between 30 min and 1.5 hour. The homogenisation is made at a temperature between 0°C and 10°C, preferably between 2°C and 6°C, more preferably at about 4°C.

The mix is then considered as stable for a couple of hours, at least up to 16 hours from the mixing, preferably up to 24 hours therefrom.

It means that the interaction matrix is representative of the interactions that should occurred between the microorganisms for a stabilized mix at 4°C.

Other interaction matrices may be produced that are representative of other mixing conditions.

The individual profiles $\{a_x(j)\}_j$ with j=1... m of the samples x are known or obtained from a sequencer profiling the samples x. Hence, reference linear-predicted mix profiles $\{i_{ref}(j)\}_j$ are also known by using the above linear formula I = P * A.

The mix profiles of the reference mix products 'ref', referred as to reference true mix profiles $\{r_{true}(j)\}_j$, are also known or obtained from a sequencer profiling the reference mix products 'ref'.

Reference predicted mix profiles $\{r_{pred}(j)\}_j$ correspond to a matrix product between the reference linear-predicted mix profiles $\{i_{ref}(j)\}_j$ and the square interaction matrix W (in the process of being learnt): $R_{pred} = I_{ref} * W$ or $\{r_{pred}(j)\}_j = \{i_{ref}(j)\}_j * W$ for a single reference mix product 'ref'.

The machine learning seeks to minimize an error in the prediction of the reference mix profiles. In other words, it seeks to minimize a formula that is based on a difference between the reference true mix profiles

$$R_{true} = \begin{bmatrix} \{r_{true-1}(j)\}_j \\ \{r_{true-N}(j)\}_j \end{bmatrix}$$

and the corresponding reference linear-predicted mix profiles

$$R_{pred} = \begin{bmatrix} \{r_{pred-1}(j)\}_j \\ \{r_{pred-N}(j)\}_j \end{bmatrix} = I_{ref} * W.$$

'pred-i' and 'true-i' reference the predicted and true reference mix profiles corresponding to the same reference mix product 'i', respectively. 'N' represents the number of reference mix products considered.

The training data for the machine learning are $I_{ref}$ and $R_{true}$.

In some embodiments, the formula to minimize is the residual vectors

$$\{r_{true-i}(j)\}_j - \{r_{pred-i}(k)\}_k = \{r_{true-i}(j)\}_j - \{i_{ref}(k)\}_k * W,$$

or the residual matrix $R_{true} - R_{pred} = R_{true} - I_{ref} * W$.

Any norm may be used: L1, L2, Lp and so on. Preferably, the Sum of Squared Difference (SSD) or its derived Mean-Squared Error (MSE) may be used. Also the minimum Chi-squared method may be used alternatively.

The machine learning may then seek to solve the following convex optimization problem:

$$min\left(\frac{1}{N} * \left\|I_{ref} * W - R_{true}\right\|^2\right)$$ where $\|.\|^2$ is the MSE and N is the number of mix products considered in $R_{true}$ and $R_{pred}$.

In embodiments avoiding overfitting W, the formula adds a regularization term, preferably a Ridge, L2,-based regularization term, to said difference. In a variant a Lasso, L1,-based regularization term can be used. The Ridge approach advantageously helps having a higher number of non-zero coefficients in W, hence modelling more precisely the interactions between the profiling features.

Hence, the machine learning seeks to solve the following convex optimization problem:

$$min\left(\frac{1}{N} * \left\|I_{ref} * W - R_{true}\right\|^2 + 1 * \|W - ID\|_2\right)$$ where $\|.\|^2$ is the regularization term (preferably Ridge), ID is the identity matrix, and $\lambda$ is an hyper-parameter for the regularization weighting.

In addition, constraints may be set during the machine learning so that $R_{pred}$ has no negative relative abundancies and the sum of the relative abundancies of each reference predicted mix profile is 1. In other words, a modified matrix $R'_{pred}$ is preferably used corresponding to clipping the negative relative abundancies in $I_{ref} * W$ and then to normalizing to 1 the sum of the relative abundancies for each reference predicted mix profile, i.e. for each line in $I_{ref} *$ $W$. Modified $I_{ref} * W$ is noted $\widehat{I_{ref} * W}$. Therefore, in embodiments, the machine learning seeks to solve the following convex optimization problem:

$$min\left(\frac{1}{N} * \left\|\widehat{I_{ref} * W} - R_{true}\right\|^2 + 1 * \|W - ID\|_2\right).$$

The set of training data, let say N reference mix results, is split into two subsets, one for the optimization of the hyper-parameter $\lambda$ and the other for the optimization of W.

Various methods to optimize $\lambda$ are known, including *inter alia* a minimizing information criteria approach (for instance minimizing Akaike or Bayesian Information Criterion) or a minimizing cross-validated residual approach, that use the first subset of training data. For this optimization, W may be set by default different from ID.

For example, the MSE of the above formula with $\lambda$ varying between $10^{-5}$ and $10^3$ is computed for a training dataset and a test dataset (splitting the subset for optimization of hyper-parameter $\lambda$). The resulting MSE is as shown in **Figure 1a.**

As shown, when $\lambda$ is small, the train dataset MSE is close to 0 while the test dataset MSE is very high. In this situation, the model is overfitted.

On the other hand, when $\lambda$ is high, the model is underfitted.

$\lambda$ may therefore be chosen to minimize the MSE for the test dataset.

Once $\lambda$ is known, the second subset of training data is used to learn W by minimizing cross-validated residuals: a k-fold procedure is performed.

The subset of training data (i.e. $\{r_{true-i}(j)\}_j$ and $\{i_{ref-i}(j)\}_j$) is split into k subsets, preferably k is selected from integers 3 to 20, preferably from 4 to 10, more preferably is equal to 5.

Each of the k subsets is successively selected in a round-robin fashion (circular order) to define a test subset, while the k-1 remaining subsets define a training subset.

For each of the k rounds, the model is trained using the training subset, i.e. $$min\left(\frac{1}{N} * \left\|\widehat{I_{ref} * W} - R_{true}\right\|^2 + 1 * \|W - ID\|_2\right)$$ is solved to find W. Advantageously, all the linear-predicted mix profiles of the training subset are fed into a single matrix $I_{ref}$ (and the true mix profiles in $R_{true}$) so as to learn W in a single pass.

The learnt interaction matrix W is then checked with the test subset; the test subset is applied to the matrix-based model $R_{true} = I_{ref} * W$. A score based on any norm, for instance on the MSE $\frac{1}{N} * \left\|\widehat{I_{ref} * W} - R_{true}\right\|^2$ is obtained.

As this operation is repeated for each k test subset, k scores are obtained.

The learnt interaction matrix W corresponding to the best score (i.e. the lowest one) can then be selected to configure pool predictor 13.

Of course, other methodologies for machine learning can be used, provided a learnt interaction matrix W is obtained.

As mentioned above, pool predictor 13 outputs a final mix result matrix R' = $\begin{bmatrix} \{r'_1(j)\}_j \\ \{r'_k(j)\}_j \end{bmatrix}$ mixes

$$P = \begin{bmatrix} \{p_1(k)\}_k \\ \{p_k(k)\}_k \end{bmatrix}$$ are provided as inputs.

Mix result matrix R', i.e. the predicted mix profiles $\{r'_x(j)\}_j$, obtained by test and decision module 14 can then be used to control a process of actual producing a mix result product 19. For instance, it may be used to control, through signalling S1 and optionally S2, an actual picking and mixing of samples from the initial sample collection 10 to obtain the mix result product.

One of the predicted mix profiles in R' may be selected by decision module 14 to trigger the production of the mix result product 19.

One or more selection criteria may be used to select one of the predicted mix profiles.

The selection criteria may be stored in a file 141 in memory. Criteria may be input in the system (list 141) by an operator and mirror requirements of the mix result product to have for instance curative or treatment properties.

The criteria relate to the profiling features of the profiles. Hence, it is synonymous to a target mix profile, the constraints on profiling features being more or less loosened depending on the embodiments.

The criteria may include a diversity criterion, for instance a bacterial diversity criterion.

By "diversity" or "bacterial diversity" it is meant the diversity or variability of the complex community of microorganisms (mix or sample), e.g. measured at the level of the genus, species, genes, functions or metabolites. The diversity can be expressed with alpha-diversity parameters to describe the complex community such as richness (number of species or genera or genes observed), Shannon index, Simpson index and Inverse Simpson index; and with beta-diversity parameters to compare complex communities such as Bray-Curtis index, UniFrac index and Jaccard index.

A diversity criterion may thus represent a requirement in terms of presence (i.e. non-zero corresponding relative abundancies) of a minimum number of profiling features (e.g. bacteria genera) or of one or more predefined profiling features. The minimum number of profiling features may be considered with respect to all the m profiling features or in a variant with respect to a predefined sublist of the m profiling features. This allows the selection process to be focused on specific features for the desired mix result product 19.

A predicted mix profile of R' that satisfies the presence of the minimum number of profiling features may for instance be selected.

A diversity criterion may represent a minimum or maximum relative abundance of one or more specific profiling features. For instance, a given bacteria genus may be desired in the mix result product within at least 5% in proportion (mass) compared to the other bacteria (specified in other profiling features). The diversity criterion may also define a range to which the relative abundance of one or more specific profiling features should belong. Of course, various diversity criteria may be mixed: a minimum or maximum relative abundance for one profiling feature with a range for another feature and/or with a maximum relative abundance for a third feature. And so on.

Similarly, a relative ratio (possibly minimum and/or maximum ratio) between at least two profiling features may be used as a diversity criterion.

A predicted mix profile of R' that satisfies the minimum or maximum relative abundancies of the specific profiling features may for instance be selected.

A diversity criterion may also represent an increase in profiling feature diversity.

A diversity criterion may define a closeness or similarity to a specific target mix profile. For instance, a target mix profile may be defined when a mix result product exactly matching the target mix profile is desired. Usually, the target mix profile is provided together with a maximal value corresponding to a distance (measurement) evaluated between profiles. A mix profile is said to be close to the target mix profile when the distance between both profiles (given the measurement) is below the maximal value. The measurement may be any norm, L1, L2, ..., Lp, the SSD, the MSE, Beta-diversity indexes or any other known distance measurement between the profiling features (e.g. Bray-Curtis, Jaccard, unifrac distances or similarity measures).

The predicted mix profile of R' with the minimal distance to the target mix profile may for instance be selected.

All or part of the above-defined criteria may be combined.

The one or more selection criteria to be used to select one of the predicted mix profiles (i.e. more generally the target mix profile) are retrieved by decision module 14 and applied to R'.

The predicted mix profiles within R' may be considered successively in order.

The first predicted mix profile meeting the selection criterion or criteria may be selected for the production of the mix result product 19.

In a variant, all the predicted mix profiles of R' are evaluated with regard to the selection criterion or criteria, and the one having the best score (e.g. meeting some criteria and / or being the closest to some others) is selected.

More generally, a reverse prediction may be contemplated where the selection criterion / target mix profile is defined, for instance that corresponds to target mix products having curative properties. File 140 may define candidate sets of samples to be tested from which it is searched the "best" set given the target mix profile. The process may be iterative meaning that a first group of disparate candidate sets (i.e. with disparate mixes in terms of which samples are mixed together and with which respective proportions) can be first tested through file 140 to find the "best" one, and then another group of candidate sets in the vicinity of the "best" set (e.q. with modification of the proportions $p_x(k)$ of the samples within the "best" set and/or with the addition or deletion of only 1 or at most 2 samples to/from the set) may then be tested. Of course one or more additional iterations may be contemplated to progressively refine the "best" result set of samples given the target mix profile.

At each iteration, the predicted mix profile of R' with the minimal distance (Bray-Curtis, Jaccard, unifrac distances and so on.) to the target mix profile may for instance be selected as the "best" one. In a variant, multiple (e.g. matrix-based) predictions may be made, and the "best" candidate set is selected from the multiple (e.g. various matrices R') predicted mix profiles so obtained. A comparison between the predicted mix profiles and the target mix profile is thus conducted to choose one candidate set as the "best" one, i.e. as a target set.

Once a predicted mix profile is selected, hence the corresponding target set of samples from the initial collection is known, the process to produce the mix result product 19 starts.

Decision module 14 first retrieves the mix composition $\{p_x(k)\}_k$ corresponding to the selected predicted mix profile, i.e. retrieves the proportions $p_x(k)$ for each sample k of the target set of samples in the initial collection 10. It then signals, using S1, a selector and mixer 15 with these proportions $\{p_x(k)\}_k$. In a variant, the signal S1 may be a display to an operator: for instance the proportions $\{p_x(k)\}_k$ are displayed, on a screen, to the operator for him or her to manually perform the actual picking and mixing of samples.

Selector and mixer 15 may be a machine having mechanical access (for instance through a controlled articulated arm) to the collection 10 of samples and including a bioreactor where performing the mixing of samples.

In response to signal S1, selector and mixer 15 picks, i.e. retrieves or takes, the samples having non-zero proportions $p_x(k)$ from bank 10, takes an amount of each sample given the corresponding proportion $p_x(k)$ and a total volume or mass targeted for the mix result product 19. The taken amounts of all the samples are poured in the bioreactor where they are actually mixed.

Preferably they are homogenised during a period of 10 minutes to 3 hours, preferably between 30 min and 1.5 hour. The homogenisation is made at a temperature between 0°C and 10°C, preferably between 2°C and 8°C, more preferably at about 4°C. The mix result product is then considered as stable for a couple of hours, at least up to 16 hours from the mixing, preferably up to 24 hours therefrom.

The true mix profile 191 $\{r_{tree}(j)\}_j$ of the resulting mix result product 19 may be obtained using profiler/ sequencer 12. As it may slightly differ from the selected predicted mix profile 192 $\{r'_x(j)\}_j$, it may be used (together with the corresponding intermediary mix profile $\{i_x(j)\}_j$) as further training data in order to improve W. A new round as described above may be implemented with this sole new item of training data (or when a couple of items is obtained) where λ may be kept unchanged and W is initially set to its current value. This iterative learning of W as the platform 1 is used, advantageously refines W, hence provides better future mix profile predictions.

As mentioned above, some samples 100y-100z may be virtual. In case such a virtual sample is selected by decision module 14 (i.e. its corresponding relative abundance $p_x(k)$ in the selected predicted mix profile is not zero), there is a need to actually produce said sample from its virtual definition (i.e. the corresponding individual profile).

When decision module 14 detects such non-zero relative abundance for a virtual sample 100y-100z corresponding to a bacterial consortium, it signals, using S2, a sample generator 16 with the need of producing said artificial sample. S2 may identify the sample concerned and indicate the amount of material needed (i.e. the corresponding proportion $p_x(k)$ multiplied by the target total volume or mass for the mix result product 19).

Sample generator 16 may be a machine having mechanical access (for instance through a controlled articulated arm) to a bank of isolated strains 160 and having storage access to a file 161 defining the composition of samples in terms of mix of individual strains. Sample generator 16 also includes a bioreactor where performing the mixing of the strains.

In response to signal S2, sample generator 16 retrieves the definition of artificial samples (bacterial consortia) in terms of strains and takes the appropriate amount of each required strain from the strain bank 16 given the signalled amount of material needed. The taken amounts of all required strains are poured in the bioreactor where they are actually mixed, for instance during 30 minutes at 4°C.

In embodiments, sample generator 16 may have access to bank 10 and / or even to a bank of external samples 99. When decision module 14 detects a non-zero relative abundance for a virtual sample corresponding to an engineered or processed complex community (i.e. a mix involving a sample), it signals, using S2, sample generator 16 with the need of producing said engineered or processed sample. S2 may identify each strain and / or each sample in bank 10 and / or each external sample concerned by the mix and indicate the amount of material needed (i.e. the corresponding proportion $p_x(k)$ multiplied by the target total volume or mass for the mix result product 19).

In response to signal S2, sample generator 16 retrieves or picks the materials, pours them in the bioreactor where they are actually mixed.

Once the mix is done and stabilized, the sample has been generated, hence it is stored in the initial sample collection or bank 10 where the selector and mixer 15 can take it to actually produce mix result product 19.

Although signals S1 and S2 are described above as control signal to drive the selector and mixer 15 and the sample generator 16, one or both of them can be mere signals displayed to an operator for him or her to actually and manually perform the mixing.

**Figure 2** illustrates, using a flowchart, general steps of producing such mix result product 19, including predicting mix profiles. These steps are performed by platform 1.

At step 200, test and decision module 14 selects a set of samples from those available in the initial sample collection 11. This step may merely consist in providing or selecting one mix definition $\{p_x(k)\}_k$ from list 140.

The definition of mix 'x' is provided to pool predictor 13 for prediction.

'x' is initially set to 1 when the mix definitions in list 140 are indexed from 1 to $N_{mix}$.

At step 205, pool predictor 13 first performs a linear prediction of the mix profile to obtain an intermediary mix profile: $\{i_x(j)\}_j = \{p_x(k)\}_k * A$. This is a matrix product.

At step 210, pool predictor 13 then performs the correction of the intermediary mix profile using interaction matrix W: $\{r_x(j)\}_j = \{i_x(j)\}_j * W$. This is a matrix product.

Clipping and normalization of $\{r_x(j)\}_j$ are also performed if needed to obtain the predicted mix profile 215 for mix 'x', $\{r'_x(j)\}_j$.

At step 220, it is checked whether $\{r'_x(j)\}_j$ satisfies a selection-criterion-based condition. The condition may be defined by a single selection criterion to satisfy or by a multiplicity thereof. The condition may mirror criteria defining a complex community of microorganisms or a microbiota composition with desired curative or treatment properties.

If $\{r'_x(j)\}_j$ does not satisfy the condition, another mix is considered through step 225 (incrementing the mix index) before looping back to step 200. Of course, if all the mixes have yet been considered (test 221), the process ends without having any mix satisfying the condition. An alert message may then be issued to an operator.

If $\{r'_x(j)\}_j$ does satisfy the condition, it is selected and the process goes to step 230.

At step 230, the corresponding mix definition $\{p_{select}(k)\}_k$ is retrieved from list 140 by decision module 14 and sent to selector and mixer 15 or displayed to the operator (signal S1).

Optionally, where a virtual sample 'k' has a corresponding non-zero proportion $p_{select}(k)$, decision module 14 triggers its actual production by sample generator 16 or displays the information to the operator, through the sending of signal S2.

At step 235, the samples with corresponding non-zero $p_{select}(k)$ are retrieved by selector and mixer 15 from bank 10 and then mixed together in a bioreactor.

It results the desired mix result product 19.

Thanks to the above prediction operation, an accurate profile of a mix result product may be obtained (at least estimated) quickly without consuming material (samples).

However, the samples may disappear over time (to actually produce some products or because they deteriorate over time) while new samples may be collected from new donors. It turns out that the collection 10 may evolve over time (thus A evolves), after a mix definition is determined to produce a target mix result product. Thanks to the invention, pool predictor 13 may be configured anew with the evolved collection (A is redefined and W is learnt) and another mix definition corresponding to the evolved collection can also be determined (using the prediction of the invention) that allows a similar mix result product to be generated.

The above sequence of steps 200-235 selects the first mix in list 140 that satisfies the condition.

In a variant, a predicted mix profile may be estimated by pool predictor 13 for all the mixes defined in list 140 before checking the condition at step 220 to find the "best" mix, i.e. set of samples.

This approach, as illustrated in **Figure 3,** seeks to find a mix definition $\{p_x(k)\}_k$ (i.e. a set of samples of collection 10) from a target mix profile. Such determination of a target mix definition (i.e. a target set of samples) may take place in a production process as described now. Indeed, once the target mix definition $\{p_x(k)\}_k$ for the target mix profile has been obtained by test and decision module 14, it may be used to control a process of actual producing a mix result product 19 as described above: decision module 14 can send signal S1, and optionally signal S2, to control modules 15 and 16 in the production process or inform the operator of the operations to conduct.

**Figure 3** illustrates, using a flowchart, general steps of producing such mix result product 19 given the target mix profile. These steps are performed by platform 1.

At step 300, the target mix profile $\{r'_x(j)\}_j$ corresponding to a desired mix result product is set in file 141.

The target mix profile may be a profile with well-defined values for the profiling features, but also a profile defining more loosened values, for instance a minimum relative abundance for one or more profiling features, a maximum relative abundance for one or more profiling features, a range for the relative abundance of one or more profiling features, a minimum number of non-zero relative abundances in the profile or in a predefined subset of profiling features, predefined profiling features with non-zero relative abundancies, a defined ratio between relative abundancies of two or more profiling features, and so on.

The target mix profile may be defined in file 141 in such a way test and decision module 14 is able to feed pool predictor 13 with this target mix profile as $\{r'(j)\}_j$ (output of the model).

At step 305, a group of candidate sets of samples from collection 10 are obtained. They may be predefined.

Random selection of samples from the collection can be performed as well as random selection of respective mix proportions $p_x(k)$.

The number of samples to mix may be selected within a range of authorized numbers, e.g. 2 to 1000 samples, preferably 3 to 100. In practice, 3 to 10 is easily handled. Of course, computer-implemented method according to the invention makes it possible at low cost to perform predictions for a higher number of samples mixed together.

The mix proportions may be selected from a group of predefined proportions (given the number of samples mixed together as the total of proportions must be 100%).

An initial group of candidate sets may be randomly formed, usually resulting into very disparate sets of samples. Another group of candidate sets may be formed given one or more know sets (e.g. determined as "best" sets in a previous iteration of the process of **Figure 3**). The other group of candidate sets may for instance include other candidate sets that depart from the know set or sets by only different mix proportions $p_x(k)$ and/or by a limited number of different samples (e.g. only 1 or 2 different samples).

The candidate sets are defined in file 140 in such a way test and decision module 14 is able to feed pool predictor 13 with these candidate mixes (lists of $\{p_x(k)\}_k$).

Next, steps 205, 210, 215 described above are performed in order to predict one or more mix profiles $\{r_x(j)\}_j$ for the candidate sets/mixes. Thanks to the matrix-based approach, the mix profiles of multiple candidate sets (possibly all) may be simultaneously predicted, where P thus includes multiple or all $\{p_x(k)\}_k$ from list 140..

Step 310 checks whether all the candidate sets/mixes have been processed (test 216). In the negative, the next mix definition is considered through step 225.

Next, at step 315, the predicted mix profiles are compared to the target mix profile (selection criterion) with a view to choose one candidate set as the target set.

For instance, a distance, e.g. a Bray-Curtis distance or a Jaccard distance or an unifrac distance or a combination thereof, is calculated for each predicted mix profile (hence for each candidate set).

Step 320 determines the closest predicted mix profile or profiles to the target mix profile given the considered distance. Preferably, the closest one is determined.

A distance margin may be implemented to guarantee the closest predicted mix profile is close enough to the target mix profile. In this situation, the closest predicted mix profile must satisfy the margin, meaning its calculated distance must be less that this margin. If no predicted mix profile satisfies the test, the process ends and an alert message may be issued to an operator.

Otherwise predicted mix profile $\{r'_{select}(j)\}_j$ 325 corresponding to target set/mix composition 'select' has been determined when entering step 230 described above.

In some embodiments (not shown in the Figure), this target set/mix composition may be used to define a new group of candidate sets as explained above in order to perform another (even more) round of the process and refine the target set/mix composition to be used at step 230.

At step 230, the mix definition 'select' is sent to selector and mixer 15 or to the operator (signal S1).

Optionally, where a virtual sample 'k' has a corresponding non-zero proportion $p_{select}(k)$, decision module 14 triggers its actual production by signalling sample generator 16 or informing the operator, through the sending of signal S2.

At step 235, the samples with corresponding non-zero $p_{select}(k)$ are retrieved or picked, e.g. by selector and mixer 15 from bank 10, and then mixed together in a bioreactor.

It results the desired mix result product 19.

A Fecal Microbiota Transfer (FMT) product and more generally a Microbiome Ecosystem Therapy product can be built from several samples. The mixing strategy as defined by the present invention allows the diversity of the final FMT product to be efficiently improved compared to a mono sample strategy, furthermore without wasting material.

Platform 1 described above with reference to **Figure 1** comprises several modules that are under the control of a central computer. For instance pool predictor 13 and test and decision module 14 are implemented in the central computer while sequencer 12, selector and mixer 15, sample generator 16 and bank 10 are separate machines connected to the central computer.

The description above mainly uses a matrix-based prediction model, in particular a square interaction matrix. Alternatives to the latter include deep learning models, such as neural networks made of multiple layers of parameterized differentiable nonlinear modules that can be trained or learnt by backpropagation.

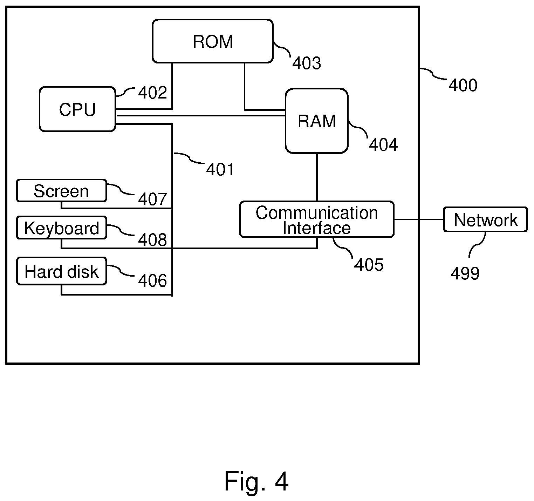

**Figure 4** schematically illustrates a computer device 400 managing the production platform 1. Computer device 400 may for instance implement pool predictor 13 and test and decision module 14 and may control sequencer 12, selector and mixer 15 and sample generator 16 via adapted signalling (S1 and S2).

The computer device 400 is configured to implement at least one embodiment of the present invention. The computer device 400 may preferably be a device such as a micro-computer, a workstation or a light portable device. The computer device 400 comprises a communication bus 401 to which there are preferably connected:

- a central processing unit 402, such as a microprocessor, denoted CPU;
- a read only memory 403, denoted ROM, for storing computer programs for implementing the invention;
- a random-access memory 404, denoted RAM, for storing the executable code of methods according to embodiments of the invention as well as the registers adapted to record variables and parameters necessary for implementing methods according to embodiments of the invention;
- a communication interface 405 connected to a network 499 in order to communication with a user or operator device and/or with other devices of platform 1, for instance sequencer 12, selector and mixer 15 and sample generator 16; and
- a data storage means 406 such as a hard disk or a flash memory, for storing computer programs for implementing methods according to one or more embodiments of the invention as well as any data necessary for embodiments of the invention, including *inter alia* individual sample profiles (i.e. collection 11), lists 140 and 141.

Optionally, the computer device 400 may also include a screen 407 serving as a graphical interface with an operator, for instance to configure the platform by means of a keyboard 408 or any other pointing means (e.g. defining lists 140, 141 as well as collection 11 and virtual samples 110y-z) and / or to display the results of the prediction process or of the reverse operation, for instance to display the target mix definition $\{p_{select}(j)\}_j$.

The computer device 400 may be optionally connected to various peripherals useless for the present invention, the sequencer 12, each being connected to an input/output card (not shown).

Preferably the communication bus provides communication and interoperability between the various elements included in the computer device 400 or connected to it. The representation of the bus is not limitative and in particular the central processing unit is operable to communicate instructions to any element of the computer device 400 directly or by means of another element of the computer device 400.

The executable code may optionally be stored either in read only memory 403, on the hard disk 406 or on a removable digital medium (not shown). According to an optional variant, the executable code of the programs can be received by means of the communication network 499, via the interface 405, in order to be stored in one of the storage means of the computer device 400, such as the hard disk 406, before being executed.

The central processing unit 402 is preferably adapted to control and direct the execution of the instructions or portions of software code of the program or programs according to the invention, which instructions are stored in one of the aforementioned storage means. On powering up, the program or programs that are stored in a nonvolatile memory, for example on the hard disk 406 or in the read only memory 403, are transferred into the random-access memory 404, which then contains the executable code of the program or programs, as well as registers for storing the variables and parameters necessary for implementing the invention.

## EXPERIMENTAL RESULTS

### Scope of the experiments

The purpose of the experiments was to investigate the efficiency of the interaction matrix W, including its machine learning procedure as proposed, to predict the mix profile of a mix of microbiota samples (Experiment 1) and to determine a mix composition given a target mix profile (Experiment 2).

### Experiment 1 - Protocol

Initial sample collection 10 was considered. Corresponding initial profile collection 11 was obtained by sequencing, using a 16S based microbiota taxa profiling, each of the microbiota samples. Hence, 131 taxa (at genus level) were evaluated as profiling features.

Next, mixing of the samples were realized. Each mix product was a combination of three to six samples with respective ratios. The mixing was performed at 4°C and the mix homogenized during 30 min to 1h30 after mixing. The mix products were sequenced, using the same 16S based microbiota taxa profiling, during their stable state (i.e. during the hours following the homogenization, less than 16h from the mixing).

A k-fold cross-validation strategy was employed with k=5 to configure pool predictor 13, i.e. to learn λ and interaction matrix W. The k-fold strategy ensured that none of the observations was used as training data and as test set during the a same evaluation.

The modelization method as described in the material and methods section was tested and applied at three different taxonomic ranks: species, genus, family and order. However, the species level datasets were very sparse, so it was excluded from the testing procedure. Starting at the genus level, the assignation tables were rich enough to allow the analyses, so the less resolved levels (family, order) were deduced from the genus tables, only for visualization purposes when needed, but not used as is in the modelization procedure. The main reason is that it is not possible to deduce from a taxa level used in the training, the composition of a higher resolved level, and having the genera information is important in our application perspective.

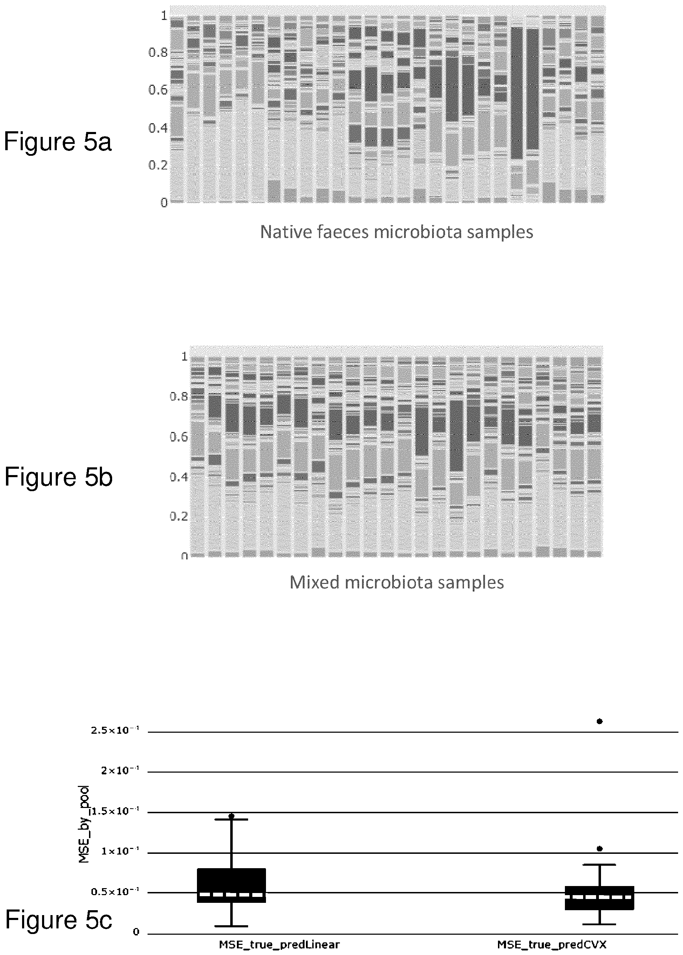

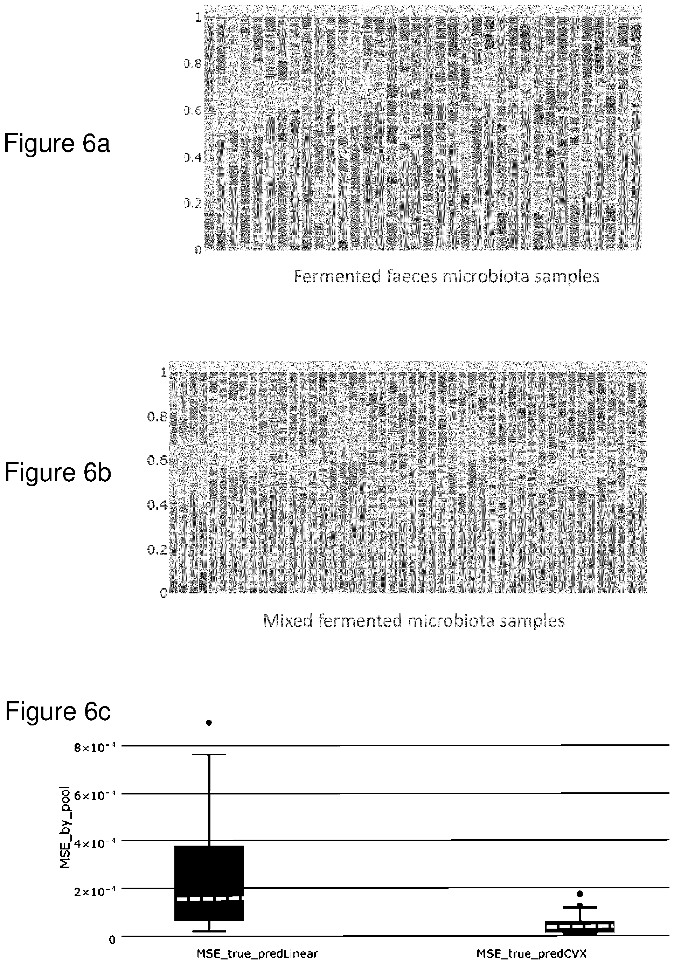

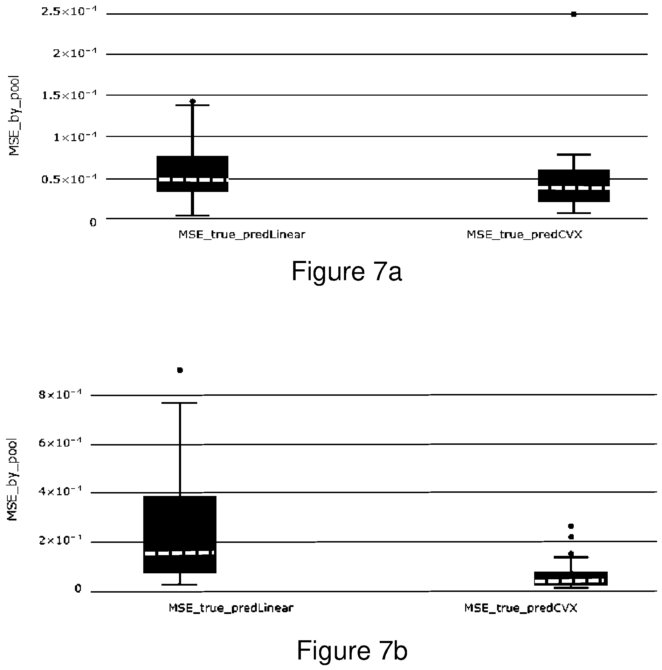

We have trained the models for native samples only **(Figures 5)** and for fermented samples **(Figures 6)** separately as well as both combined **(Figures 7).** The MSE was used to quantify the quality of the modelization when applied on the data. The MSE were systematically compared between the machine learning model and the linear model (the one providing the naïve predictions).

## Experiment 1 - Results

**Figure 5a** illustrates the initial profile collection 11 corresponding to an initial sample collection 10 comprising only native faeces microbiota samples. 27 microbiota samples were considered. Their individual profiles are depicted in the Figure.

**Figure 5b** illustrates the mix profiles of 24 mix products mixing three to six microbiota samples from amongst the 27 microbiota samples of **Figure 5a,** with respective ratios or proportions. The mix definitions $\{p_x(k)\}_k$ are saved.

**Figure 5c** illustrates, on the left side, the error resulting from a linear prediction of the mix profiles given the mix definitions $\{p_x(k)\}_k$ and the individual sample profiles $\{a_x(j)\}_j$. The linear prediction corresponds to the sole step 205: I = A * P.

The Figure also illustrates, on the right side, the error resulting from the prediction according to the invention (steps 205 and 210), i.e. involving the interaction matrix W. W was machine-learned using only the sample and mix profiles of **Figures 5a** and **5b** (native samples) with the k-fold cross-validation strategy.

The model-based method of the invention returns a better performance than the linear method for the native dataset.

**Figure 6a** illustrates the initial profile collection 11 corresponding to an initial sample collection 10 comprising only fermented faeces microbiota samples. 36 microbiota samples were considered. Their individual profiles are depicted in the Figure.

**Figure 6b** illustrates the mix profiles of 48 mix products mixing three to six microbiota samples from amongst the 36 microbiota samples of **Figure 6a,** with respective ratios or proportions. The mix definitions $\{p_x(k)\}_k$ are saved.

**Figure 6c** illustrates, on the left side, the error resulting from a linear prediction of the mix profiles given the mix definitions $\{p_x(k)\}_k$ and the individual sample profiles $\{a_x(j)\}_j$. The linear prediction corresponds to the sole step 205: I = A * P.

The Figure also illustrates, on the right side, the error resulting from the prediction according to the invention (steps 205 and 210), i.e. involving the interaction matrix W. W was machine-learned using only the sample and mix profiles of **Figures 6a** and **6b** (fermented samples) with the k-fold cross-validation strategy.

The model-based method of the invention returns a dramatically better performance than the linear method for the fermented dataset (median MSE is 5x lower with the ML model predictions).

For **Figures 7a** and **7b,** interaction matrix W was machine-learned using both sample and mix profiles of **Figures 5a, 5b, 6a** and **6b** (i.e. native and fermented samples) as training data. Again, the k-fold cross-validation strategy was used.

**Figure 7a** shows the result when the dataset of **Figures 5a, 5b** (i.e. native samples and mixes thereof) are applied to pool predictor 13 so configured.

The left side of the Figure depicts the error resulting from a linear prediction of the mix profiles given the mix definitions $\{p_x(k)\}_k$ and the individual sample profiles $\{a_x(j)\}_j$.

The right side depicts the error resulting from the prediction according to the invention (steps 205 and 210), i.e. involving the interaction matrix W so learnt.

As for the single dataset model, the combined datasets model improves slightly the estimation when applied to the native dataset.

**Figure 7b** shows the result when the dataset of **Figures 6a, 6b** (i.e. fermenter samples and mixes thereof) are applied to pool predictor 13 so configured.

The left side of the Figure depicts the error resulting from a linear prediction of the mix profiles given the mix definitions $\{p_x(k)\}_k$ and the individual sample profiles $\{a_x(j)\}_j$.

The right side depicts the error resulting from the prediction according to the invention (steps 205 and 210), i.e. involving the interaction matrix W so learnt.

As for the single dataset model, the combined datasets model improves dramatically the estimation when applied to the fermented dataset (median MSE is 4x lower with the ML model predictions).

**Experiment 1 - Discussion and Conclusion**

In all cases, the model-based prediction improves the naïve (linear) methods estimation. It is especially important for the fermented dataset where the naïve approach does not perform well, especially for some groups of taxa. The model-based correction approach was more efficient, probably as there were more room for improvement. If more data are added to train the model, one can assume that the overall performances and the robustness will improve. The training method, also part of this invention, allows such a model evolution.

**Experiment 2 - Protocol**

In this experiment, the interaction matrix W learned using native and fermented samples (i.e. W of **Figures 7a** and **7b**) is used.

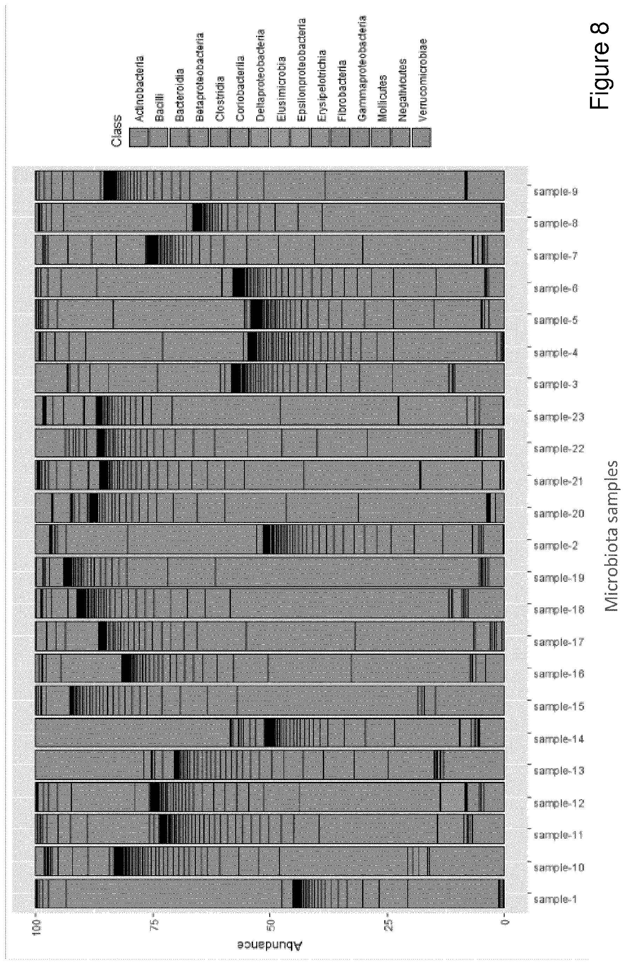

Another collection of samples was considered for this experiment. It is made of 23 samples. Corresponding collection of profiles was obtained by the same sequencing of each of the 23 microbiota samples: using a 16S based microbiota taxa profiling where 131 taxa (at genus level) were considered as profiling features. **Figure 8** illustrates the profile collection (at class level).

Pool predictor 13 was used to generate 160 mixes with different input microbiota samples (mixing 2 to 4 samples out of 23) with different mixing conditions. Four rounds (exp_1 to exp_4) of predictions were made, in which eight different sets of samples (chunk_1 to chunk_8) were considered with five different sets of proportions (Mix1 to Mix5).

Each generated predicted mix profile can then be identified by a triplet (i, j, k) where i=1...4 (exp), j=1...8 (chunk) and k=1...5 (Mix), and a corresponding name "exp_i-chunk_**j**-Mix**k**"

The different sets of proportions (in %) were predefined as follows (depending on the number of samples in the mix).

**Table 1: sample proportions in mixes of 4 samples**

|      | Sample 1 | Sample 2 | Sample 3 | Sample 4 |
|------|----------|----------|----------|----------|
| Mix1 | 10       | 10       | 20       | 60       |
| Mix2 | 20       | 20       | 20       | 40       |
| Mix3 | 10       | 20       | 30       | 40       |
| Mix4 | 20       | 30       | 20       | 30       |
| Mix5 | 25       | 25       | 25       | 25       |

**Table 2: sample proportions in mixes of 3 samples**

|      | Sample 1 | Sample 2 | Sample 3 |
|------|----------|----------|----------|
| Mix1 | 10       | 20       | 70       |
| Mix2 | 20       | 20       | 60       |
| Mix3 | 10       | 50       | 40       |
| Mix4 | 10       | 10       | 80       |
| Mix5 | 33       | 33       | 34       |

**Table 3: sample proportions in mixes of 2 samples**

|      | Sample 1 | Sample 2 |
|------|----------|----------|
| Mix1 | 10       | 90       |
| Mix2 | 20       | 80       |
| Mix3 | 30       | 70       |
| Mix4 | 40       | 60       |
| Mix5 | 50       | 50       |

The different candidate sets of samples were defined as follows.

**Table 4: compositions of candidate mixes**

| | | Sample 1 | Sample 2 | Sample 3 | Sample 4 |
|---|---|---|---|---|---|
| exp_1 | chunk_1 | sample-7 | sample-14 | sample-17 | sample-20 |
| exp_1 | chunk_2 | sample-2 | sample-4 | sample-11 | sample-22 |
| exp_1 | chunk_3 | sample-5 | sample-6 | sample-23 | |
| exp_1 | chunk_4 | sample-1 | sample-3 | | |
| exp_1 | chunk_5 | sample-9 | sample-21 | | |
| exp_1 | chunk_6 | sample-16 | sample-19 | | |
| exp_1 | chunk_7 | sample-8 | sample-13 | sample-15 | |
| exp_1 | chunk_8 | sample-10 | sample-12 | sample-18 | |
| exp_2 | chunk_1 | sample-9 | sample-12 | sample-15 | |
| exp_2 | chunk_2 | sample-3 | sample-5 | sample-16 | |
| exp_2 | chunk_3 | sample-6 | sample-8 | | |
| exp_2 | chunk_4 | sample-7 | sample-18 | sample-23 | |
| exp_2 | chunk_5 | sample-13 | sample-19 | sample-22 | |
| exp_2 | chunk_6 | sample-1 | sample-2 | sample-21 | |
| exp_2 | chunk_7 | sample-4 | sample-10 | sample-14 | |
| exp_2 | chunk_8 | sample-11 | sample-17 | sample-20 | |
| exp_3 | chunk_1 | sample-6 | sample-10 | sample-14 | sample-16 |
| exp_3 | chunk_2 | sample-1 | sample-5 | sample-11 | |
| exp_3 | chunk_3 | sample-3 | sample-4 | sample-7 | |
| exp_3 | chunk_4 | sample-9 | sample-19 | sample-22 | |
| exp_3 | chunk_5 | sample-2 | sample-12 | | |
| exp_3 | chunk_6 | sample-15 | sample-20 | | |
| exp_3 | chunk_7 | sample-13 | sample-21 | | |
| exp_3 | chunk_8 | sample-8 | sample-17 | sample-18 | sample-23 |
| exp_4 | chunk_1 | sample-6 | sample-9 | sample-15 | |
| exp_4 | chunk_2 | sample-2 | sample-18 | | |
| exp_4 | chunk_3 | sample-3 | sample-5 | sample-12 | |
| exp_4 | chunk_4 | sample-1 | sample-10 | sample-14 | sample-19 |
| exp_4 | chunk_5 | sample-16 | sample-21 | sample-22 | sample-23 |
| exp_4 | chunk_6 | sample-4 | sample-8 | | |
| exp_4 | chunk_7 | sample-13 | sample-17 | sample-20 | |
| exp_4 | chunk_8 | sample-7 | sample-11 | | |

For the present experiment, mix "exp_1-chunk_7-Mix5", i.e. a mix made of 33% of sample-8, 33% of sample-13 and 34% of sample-15, was considered as a target mix. Its predicted mix profile was used as a target mix profile.

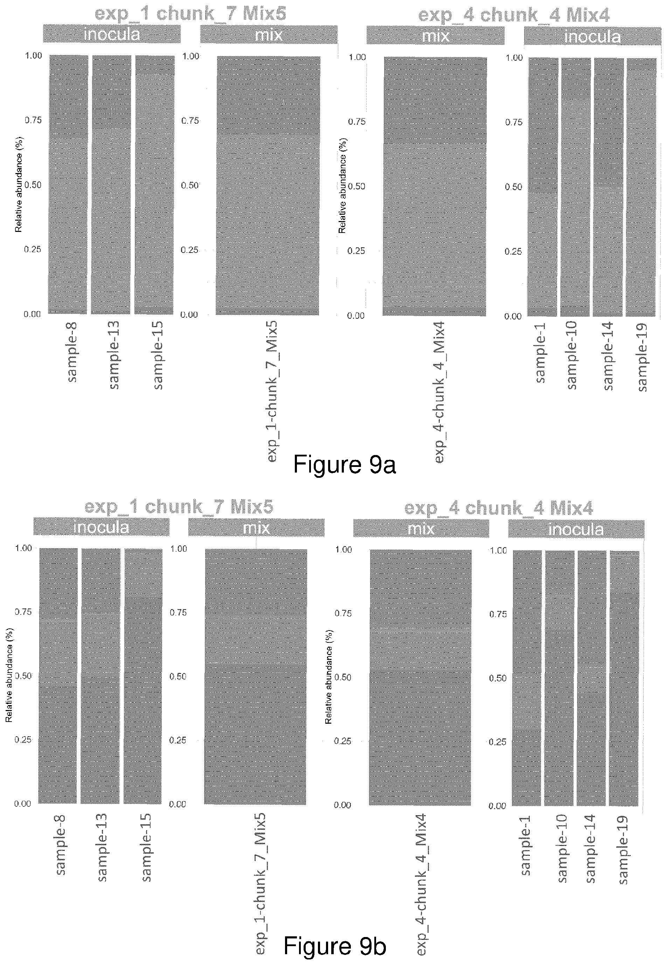

The similarity between all the proposed mixes "exp_i-chunk_**j**-Mix**k**" and the target mix "exp_1-chunk_7-Mix5" was evaluated using the Bray-Curtis index at the genus level **(Figure 9).**

The Bray-Curtis index is forced to 0 when at least two samples of the mix considered come from the same donor. This was to avoid identifying mixes having the same samples.

Mix "exp_1-chunk_7-Mix5" and the most similar mixes for both metrics were actually mixed, and the resulting products were sequenced using the same sequencing technique with a view of comparing their composition at the phylum and family levels.

**Experiment 2 - Results**

Table 5 below shows the 15 highest Bray-Curtis similarities (based on the genera abundances) calculated from the predicted mix profiles. The Bray-Curtis similarity equals 1 - Bray-Curtis dissimilarity measure.

**Table 5: Bray-Curtis similarities to exp_1-chunk_7-Mix5**

| | |
|---|---|
| exp_4-chunk_4-Mix4 | 0.861 |
| exp_4-chunk_4-Mix5 | 0.852 |
| exp_4-chunk_4-Mix2 | 0.848 |
| exp_4-chunk_4-Mix3 | 0.835 |
| exp_4-chunk_4-Mix1 | 0.805 |
| exp_4-chunk_2-Mix4 | 0.772 |
| exp_4-chunk_2-Mix3 | 0.763 |
| exp_1-chunk_8-Mix5 | 0.762 |
| exp_4-chunk_2-Mix5 | 0.749 |
| exp_4-chunk_2-Mix2 | 0.747 |
| exp_1-chunk_8-Mix2 | 0.745 |
| exp_3-chunk_1-Mix4 | 0.743 |
| exp_3-chunk_1-Mix5 | 0.743 |
| exp_1-chunk_8-Mix3 | 0.742 |
| exp_2-chunk_7-Mix5 | 0.734 |

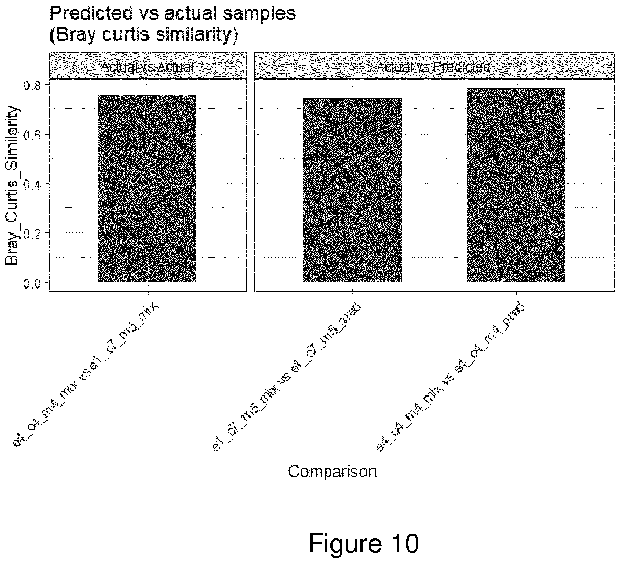

**Figure 9a** shows the true profiles of the samples composing exp_1-chunk_7-Mix5 and the closest mix, exp_4-chunk_4-Mix4 (made of 20 % of sample-1, 30% of smple-10, 20% of sample-14 and 30% of sample 19), as well as their true mix profiles, at phylum level.
**Figure 9b** illustrates the same comparison at family level.
Although the initial samples have very different profiles between exp_1-chunk_7-Mix5 and exp_4-chunk_4-Mix4, the final products have very similar mix profiles at both phylum and family levels.
Table 6 and **Figure 10** show the Bray-Curtis similarity (at genus level) results of the comparisons between exp_1-chunk_7-Mix5 and the closest mix, exp_4-chunk_4-Mix4, as well as their true mix profiles.

**Table 6: Bray-Curtis similarity (genus level) between predicted and actual mixes**

| Sample_1 | Sample_2 | Bray-Curtis similarity | Type |
|---|---|---|---|
| exp_4-chunk_4-Mix4 real mix | exp_1-chunk_7-Mix5 real mix | 0.753 | Actual vs Actual |
| exp_4-chunk_4-Mix4 real mix | exp_4-chunk_4-Mix4 predicted | 0.783 | Actual vs Predicted |
| exp_1-chunk_7-Mix5 real mix | exp_1-chunk_7-Mix5 predicted | 0.739 | Actual vs Predicted |

Although the initial samples of the two mixes have very different profiles, the final products have very similar mix profiles at genus level according to the Bray-Curtis distance metric. Table 6 and **Figure 10** shows a similarity gap between an actual mix and its prediction. This however does not impact substantially the similarity between the actual mixes, which similarity remains at an acceptable level.

**Experiment 2 - Discussion and Conclusion**

The experiment 2 shows that the prediction tool can be used iteratively to predict mixes very close to a target sample (Bray-Curtis similarity at the genus level greater than 0.86), and to select corresponding samples and mix proportions for the in vitro experience phase.

It also illustrates that two mixes produced according to the prediction recipe are actually very close together (Bray-Curtis similarity at the genus level greater than 0.75). This demonstrates the performances of the prediction tool, and its applicability in a real life context.

Although the present invention has been described herein above with reference to specific embodiments, the present invention is not limited to the specific embodiments, and modifications will be apparent to a skilled person in the art which lie within the scope of the present invention.

Many further modifications and variations will suggest themselves to those versed in the art upon referring to the foregoing illustrative embodiments, which are given by way of example only and which are not intended to limit the scope of the invention, that being determined solely by the appended claims. In particular, the different features from different embodiments may be interchanged, where appropriate.

In the claims, the word "comprising" does not exclude other elements or steps, and the indefinite article "a" or "an" does not exclude a plurality. The mere fact that different features are recited in mutually different dependent claims does not indicate that a combination of these features cannot be advantageously used.

**Claims**

1. A computer-aided method of predicting a mix composition resulting from the mixing of complex microorganism community samples belonging to an initial sample collection, the method comprising:

   predicting, using a linear approach, an intermediary mix profile for a mix of selected complex microorganism community samples, and
   correcting the intermediary mix profile into a predicted mix profile, using an interaction model learnt from reference linear-predicted mix profiles and corresponding reference true mix profiles.

2. The method of Claim 1, wherein the predicted mix profile is used to control actual picking and mixing of complex microorganism community samples from the initial sample collection to obtain a mix result product.

3. The method of Claim 1 or 2, wherein predicting the intermediary mix profile includes computing a matrix product between a first matrix defining the mix in terms of proportions of the complex microorganism community samples of the initial sample collection and a second matrix defining the individual profiles of the complex microorganism community samples.

4. The method of any of Claims 1 to 3, wherein correcting the intermediary mix profile includes computing a matrix product between a matrix representing the intermediary mix profile and a square interaction matrix of the learnt interaction model.

5. A computer-aided method of determining a set of complex microorganism community samples in an initial sample collection given a target mix profile representing a target mix result product, the method comprising:

   selecting candidate sets of complex microorganism community samples from the initial sample collection,
   for each candidate set selected, using the prediction method of Claim 1 to predict a mix profile resulting from the mixing of the samples of the selected candidate set,
   comparing the predicted mix profiles to the target mix profile to choose one candidate set as the target set.

6. The method of Claim 5, wherein the target set of samples is used to control actual picking and mixing of complex microorganism community samples from the initial sample collection to obtain a mix result product function of the target mix profile.

7. The method of Claim 5 or 6, wherein comparing the predicted mix profiles to the target mix profile includes computing a distance between each predicted mix profile and the target mix profile and selecting, as target set, the candidate set having the lowest distance.

8. The method of any of Claims 1 to 7, wherein a profile of a complex community of microorganisms includes relative abundancies of profiling features in the complex community of microorganisms.

9. The method of any of Claims 1 to 8, wherein profiling features forming a profile of a complex community of microorganisms include one or more features selected from the group consisting of taxa, genes, antibiotic resistance genes, functions, metabolite traits, and metabolite and protein production, preferably include taxa.

10. The method of any of Claims 1 to 9, wherein a profile of a complex community of microorganisms defines profiling features with respect to one or more microorganisms present in the complex community of microorganisms from bacteria, archaea, viruses, phage, protozoa and fungi, preferably with respect to bacteria and/or archaea, and/or. defines profiling features that specify relative abundances of microorganisms considered at one or more taxonomic levels from strains, species, genus, families and orders, preferably one or more taxonomic levels from genus, families and orders, and/or
includes relative abundancies, in the complex community of microorganisms, of bacteria and/or archaea taxa considered at a taxonomic level of genus, families and orders.

11. The method of any of Claims 1 to 10, wherein the interaction model is obtained using machine learning that minimizes a formula function of a difference between
reference predicted mix profiles obtained from the reference linear-predicted mix profiles and the interaction model, and
the corresponding reference true mix profiles.

12. A method of producing a complex microorganism community product, comprising:

selecting complex microorganism community samples from an initial sample collection,
using the prediction method of Claim 1 to predict a mix profile resulting from the mixing of the selected samples,
comparing the predicted mix profile to a selection criterion, and
depending on the outcome of the comparing, actually picking and mixing the selected samples to obtain a mix result product.

13. The method of Claim 12, wherein the selection criterion includes one or more from a diversity criterion representative of an increase in profiling feature diversity, a minimum or maximum relative abundance of one or more profiling features, a non-zero relative abundance for one or more specific profiling features or for a minimum number of profiling features, a relative ratio between at least two profiling features, a closeness to a target mix profile.

14. A method of producing a complex microorganism community product having a target mix profile representing a target mix result product, comprising:

selecting, using the determining method of Claim 5, a target set of complex microorganism community samples belonging to an initial sample collection given the target mix profile, and
actually picking and mixing the microorganism community samples of the selected target set to obtain a mix result product.

15. The method of Claim 14, wherein one selected complex microorganism community sample is a virtual sample and the method further comprises actually producing an artificial complex microorganism community sample corresponding to the selected virtual sample from isolated strains.

16. A computer device comprising at least one microprocessor configured for carrying out the method of any of Claims 1 to 15.

17. A non-transitory computer-readable medium storing a program which, when executed by a microprocessor or computer system in a device, causes the device to perform the method of any of Claims 1 to 15.

Figure 1

Figure 1a

**Figure 3**

- 300 — Obtain target $\{r'_x(i)\}_i$
- 305 — Obtain candidate sets of samples
- 205 — Obtain $\{i_x(i)\}_i$ (linear prediction)
- 210 — Correct $\{i_x(i)\}_i$ to obtain $\{r'_x(i)\}_i$
- 215 — Predicted mix profile
- 310 — $x = N_{mix}$ ?
  - no → 225 — $x \leftarrow x+1$
  - yes → 315 — Calculate distances
- 320 — One closest mix?
  - no → End
  - yes → 230 — Trigger production via signals S1 and S2
- 235 — Mix samples
- 19 — Mix result product

**Figure 2**

- 200 — Select $\{p_x(k)\}_k$
- 205 — Obtain $\{i_x(i)\}_i$ (linear prediction)
- 210 — Correct $\{i_x(i)\}_i$ to obtain $\{r'_x(i)\}_i$
- 215 — Predicted mix profile
- 216 — $x = N_{mix}$ ?
  - no → 225 — $x \leftarrow x+1$
- 220 — Criterion met ?
  - no → 221 — $x = N_{mix}$ ? → End
  - yes → 230 — Trigger production via signals S1 and S2
- 235 — Mix samples
- 19 — Mix result product

26

image-only figure

Fig. 4

Figure 5a

Native faeces microbiota samples

Figure 5b

Mixed microbiota samples

Figure 5c

Figure 6a

Fermented faeces microbiota samples

Figure 6b

Mixed fermented microbiota samples

Figure 6c

Figure 7a

Figure 7b

Figure 8

Figure 9a

Figure 9b

Figure 10

Europäisches
Patentamt

European
Patent Office

Office européen
des brevets

# EUROPEAN SEARCH REPORT

Application Number

EP 21 17 2578

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2017/138007 A1 (YEDA RES AND DEV CO LTD AT THE WEIZMANN INST OF SCIENCE [IL]) 17 August 2017 (2017-08-17) * the whole document * * examples 4,9 * * figure 4 * | 1-17 | INV. C12N1/20 A61K35/74 |
| X | WO 2018/187272 A1 (GUSTO GLOBAL LLC [US]) 11 October 2018 (2018-10-11) * the whole document * * examples 1-10 * | 1-17 | |
| X | WO 2019/018580 A1 (UBIOME INC [US]) 24 January 2019 (2019-01-24) * the whole document * * figures 1-3,5-8 * * paragraphs [0070] - [0086], [0101] - [0117] * | 1-17 | |
| A,D | WO 2019/171012 A1 (MAAT PHARMA [FR]) 12 September 2019 (2019-09-12) * the whole document * * examples 1-4 * | 1-17 | TECHNICAL FIELDS SEARCHED (IPC) C12N A61K G16B |
| A | GU J.-L. ET AL: "Gut microbiota community adaption during young children fecal microbiota transplantation by 16s rDNA sequencing", NEUROCOMPUTING, vol. 206, 1 June 2016 (2016-06-01), pages 66-72, XP029674209, ISSN: 0925-2312, DOI: 10.1016/J.NEUCOM.2016.01.095 * the whole document * | 1-17 | |

-/--

The present search report has been drawn up for all claims

| Place of search | Date of completion of the search | Examiner |
|---|---|---|
| The Hague | 12 October 2021 | van de Kamp, Mart |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

## EUROPEAN SEARCH REPORT

Application Number

EP 21 17 2578

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GILBERT J. A. ET AL: "Community ecology as a framework for human microbiome research", NATURE MEDICINE, vol. 25, no. 6, 27 May 2019 (2019-05-27), pages 884-889, XP036901072, ISSN: 1078-8956, DOI: 10.1038/S41591-019-0464-9 [retrieved on 2019-05-27] * the whole document * | 1-17 | |
| A | WALTER J. ET AL: "To engraft or not to engraft: an ecological framework for gut microbiome modulation with live microbes", CURRENT OPINION IN BIOTECHNOLOGY, vol. 49, 1 February 2018 (2018-02-01), pages 129-139, XP055848363, GB ISSN: 0958-1669, DOI: 10.1016/j.copbio.2017.08.008 * the whole document * | 1-17 | |

TECHNICAL FIELDS
SEARCHED      (IPC)

The present search report has been drawn up for all claims

| Place of search | Date of completion of the search | Examiner |
|---|---|---|
| The Hague | 12 October 2021 | van de Kamp, Mart |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 17 2578

This annex lists the patent family members relating to the patent documents cited in the above-mentioned European search report.
The members are as contained in the European Patent Office EDP file on
The European Patent Office is in no way liable for these particulars which are merely given for the purpose of information.

12-10-2021

| Patent document cited in search report | | Publication date | Patent family member(s) | | Publication date |
|---|---|---|---|---|---|
| WO 2017138007 | A1 | 17-08-2017 | EP | 3414346 A1 | 19-12-2018 |
| | | | EP | 3708675 A1 | 16-09-2020 |
| | | | ES | 2771223 T3 | 06-07-2020 |
| | | | US | 2019142875 A1 | 16-05-2019 |
| | | | US | 2021260139 A1 | 26-08-2021 |
| | | | WO | 2017138007 A1 | 17-08-2017 |
| WO 2018187272 | A1 | 11-10-2018 | CA | 3058943 A1 | 11-10-2018 |
| | | | EP | 3606325 A1 | 12-02-2020 |
| | | | US | 2020027524 A1 | 23-01-2020 |
| | | | WO | 2018187272 A1 | 11-10-2018 |
| WO 2019018580 | A1 | 24-01-2019 | AU | 2018302175 A1 | 13-02-2020 |
| | | | CN | 111247598 A | 05-06-2020 |
| | | | EP | 3655971 A1 | 27-05-2020 |
| | | | JP | 2020528285 A | 24-09-2020 |
| | | | KR | 20200051587 A | 13-05-2020 |
| | | | SG | 11202000340S A | 27-02-2020 |
| | | | WO | 2019018580 A1 | 24-01-2019 |
| WO 2019171012 | A1 | 12-09-2019 | AU | 2019229721 A1 | 15-10-2020 |
| | | | CA | 3091626 A1 | 12-09-2019 |
| | | | CN | 111836631 A | 27-10-2020 |
| | | | EP | 3762001 A1 | 13-01-2021 |
| | | | FR | 3078627 A1 | 13-09-2019 |
| | | | JP | 2021517131 A | 15-07-2021 |
| | | | KR | 20200130367 A | 18-11-2020 |
| | | | US | 2020405776 A1 | 31-12-2020 |
| | | | WO | 2019171012 A1 | 12-09-2019 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016170285 A
- WO 2017103550 A
- WO 2019171012 A1
- WO 2016170285 A1
- WO 2017103550 A1